# EUROPEAN PATENT APPLICATION

(11) **EP 4 723 813 A1**
(43) Date of publication of application: **08.04.2026**
(21) Application number: 25190063.5
(22) Date of filing: 17.07.2025
(51) Int. Cl.: H05K 1/189, A61B 8/00

(54) **MEDICAL TOOL COMPRISING CONDUCTIVE LAYERS**

(30) Priority: 18.07.2024 US 202463673079 P; 07.07.2025 US 202519261961
(71) Applicant: Biosense Webster (Israel) Ltd., 2066717 Yokneam (IL)
(72) Inventor: RODRIGUEZ, Jasson, 2066717 Yokneam (IL); ATIAS, Meiron, 2066717 Yokneam (IL); HITZEROTH, Matthew, 2066717 Yokneam (IL); GOVEA, Michael, 2066717 Yokneam (IL)
(74) Representative: Carpmaels & Ransford LLP

(57) **Abstract**

Disclosed herein are medical devices configured and sized to be positioned within a subject. Medical devices may comprise a flexible circuit comprising at least two conductive layers. A first conductive layer may be disposed on a first surface of the flexible circuit and a second conductive layer is disposed on a second surface opposite the first surface such that electrical energy is capable of being conducted through the flexible circuit to the first conductive layer and the second conductive layer. Medical devices may comprise an electro acoustic module (EAM) disposed at or adjacent a distal region of the flexible circuit, the EAM coupled to the flexible circuit to define a distal tip of the medical device. The at least two conductive layers create trace routes via the flexible circuit to the EAM.

## Description

### PRIORITY

This application claims priority to and the benefit of U.S. Prov. App. No. 63/673,079, filed July 18, 2024.

### INCORPORATION BY REFERENCE

This application also incorporates by reference herein the entire disclosures of the following applications, which are incorporated by reference herein for all purposes: U.S. Prov. App. No. 63/673,079, filed July 18, 2024; U.S. Pat. Appl. Ser. No. 18/608,646 filed March 18, 2024; PCT/US2019/061228 filed November 13, 2019; U.S. Prov. App. No. 62/760,784 filed November 13, 2018; WO2018/017717, published 1/25/2018; US20220401070A1; and WO 2018/182836, published 10/4/2018.

All publications and patent applications mentioned in this specification are herein incorporated by reference to the same extent as if each individual publication or patent application was specifically and individually indicated to be incorporated by reference.

### BACKGROUND

A wide variety of intravascular medical devices are known. The medical devices may use conductive traces to communicate data sensed inside the body to a computing device outside the body. Conductive traces may break easily and be difficult to repair and/or replace. When the conductive traces break, the medical devices may become unusable and have to be replaced, which may be time consuming and costly. Improvements are needed.

### SUMMARY OF THE DISCLOSURE

It is to be understood that both the following general description and the following detailed description are exemplary and explanatory only and are not restrictive.

Medical devices may include a flexible circuit having at least two conductive layers. A first conductive layer may be disposed on a first surface of the flexible circuit and a second conductive layer may be disposed on a second surface opposite the first surface such that electrical energy may be capable of being conducted through the flexible circuit to the first conductive layer and the second conductive layer. Medical devices may include an electro acoustic module (EAM) disposed at or adjacent a distal region of the flexible circuit, the EAM may be coupled to the flexible circuit to define a distal tip of the medical device. Medical devices may include where the at least two conductive layers create trace routes via the flexible circuit to the EAM.

Medical devices may include an electro acoustic module (EAM) disposed at or adjacent a distal region of the medical device, the EAM at least partially enclosed in a material to define a distal tip of the medical device. Medical devices may include a handle disposed at or adjacent a proximal region of the medical device and spaced from the EAM. Medical devices may include a flexible circuit coupling the EAM and the handle, the flexible circuit having at least two conductive layers. A first conductive layer may be disposed on a first surface of the flexible circuit and a second conductive layer may be disposed on a second surface opposite the first surface such that electrical energy may be capable of being conducted through the flexible circuit to the first conductive layer and the second conductive layer. The at least two conductive layers may create trace routes via the flexible circuit to the EAM.

Medical devices may include a flexible circuit having at least two conductive layers. The at least two conductive layers may include a printed circuit board assembly (PCBA). Medical devices may include an electro acoustic module (EAM) disposed at or adjacent a distal region of the flexible circuit, the EAM coupled to a first side of the flexible circuit by a material to define a distal tip of the medical device. Medical devices may include an application-specific integrated circuit (ASIC) disposed at or adjacent the distal region of the flexible circuit, the ASIC coupled to a second side of the flexible circuit opposite the first side.

The disclosure herein is related to medical devices and uses thereof. The disclosure is generally related to flexible elongate members (e.g., flexible conductor bundles) that may extend along some length of the medical device. Some aspects of the disclosure describe ways that prevent the flexible elongate members from deforming, or at least minimize the degree of the deformation. In some examples, the disclosure herein is related to trying to minimize bending along the flexible elongate member at one or more locations. While bending is a type of deformation, the disclosure may also be related to trying to minimize or prevent folding or otherwise bunching of the flexible elongate member. In some examples, the disclosure herein is related to trying to maintain a configuration of the flexible elongate member, or at least maintain the configuration as closely as possible to a certain configuration to try to prevent undesirable consequences of a large extent of deformation.

In some examples, a force is applied to a flexible elongate member at a location that is proximal to a location wherein the flexible elongate member is secured to a medical tool. The applied force may help prevent the undesired deformation and/or maintain the desired configuration.

One aspect of the disclosure is a medical device that is sized and configured to be positioned within a subject, such as within a blood vessel, chamber of the heart, or other bodily lumen or space.

The medical devices herein may include a medical tool such as an ultrasound transducer in a distal region of the medical device. The medical tool may be secured (directly or indirectly) to a flexible member(s), such as flexible electronics (e.g., a flexible conductor bundle) at a first location. The flexible member may extend proximally towards (and optionally into) a handle assembly. In some examples, the disclosure is related to trying to prevent the flexible electronics from deforming to an undesirable extent and/or trying to maintain a configuration of the flexible electronics (even if there some minor degree of deformation).

The medical device may include one or more elongate shafts through which the flexible member extends. The medical device may include more than one elongate shaft through which the flexible member extends, such as an outer shaft and an inner shaft, the inner shaft extending through at least a portion of the outer shaft. An inner shaft may be movable relative to the outer shaft. An inner shaft may be independently deflectable relative to the outer shaft.

The flexible member (e.g., flexible conductor bundle) may be axially movable within the one or more elongate shafts, and may be fixed to a shaft, such as an outer shaft.

The medical device may include a tensioning member secured to one or more surfaces of the flexible member (e.g., a flexible conductor bundle) at a second location that is proximal to where the medical tool is secured to the flexible member. The second location may be within a handle assembly of the medical device, but the second location may be distal to a handle assembly. The second location may be positioned such that it is within the subject when the medical device is in use.

A tensioning member may be in operative communication (operatively coupled) with handle assembly actuator (e.g., knob, etc.) such that actuation (e.g., rotating, moving axially) of the handle actuator causes movement of the tensioning member. The actuator may also be in operative communication with the medical tool. In some embodiments, the tensioning member and the medical tool are in operable axial communication with a handle actuator such that actuation of the handle actuator causes axial movement of the ultrasound transducer and the tensioning member. Axial movement of the tensioning may apply tension to the flexible conductor bundle at the second location within the handle assembly.

A tensioning member may be axially secured to a flexible conductor bundle at a second location within a handle such that the tensioning member and flexible conductor bundle move together axially at the second location upon actuation of a handle actuator.

A tensioning member may be secured to the flexible conductor bundle such that the flexible conductor bundle is maintained in a substantially flattened configuration, at least at a location near the medical tool. The flexible conductor bundle may be maintained in a substantially flattened configuration between the first and second locations when the medical tool is retracted proximally.

A flexible member may have flat or generally flat first and second surfaces, and a tensioning member may be secured to one or both of the flat or generally flat first and second surfaces.

An actuator may also be adapted to be rotated to cause rotation of the medical tool, and wherein a tensioning member and the actuator are in operative communication such that rotation of the actuator does not cause rotation of the tensioning member.

A flexible member (e.g., flexible conductor bundle) may be secured to a printed circuit board ("PCB") in the handle assembly, and optionally at a location that is proximal to the printed circuit board.

A medical device inner shaft may be in operable communication with a second handle actuator such that the inner shaft is deflectable upon actuation of the second handle actuator.

The medical device may include a flexible electronics (e.g., conductor bundle) flattening member secured to one or more surfaces of the flexible electronics at a second location, wherein the second location may be in a handle assembly. The flexible electronics flattening member and the medical tool may be in operable axial communication with a handle actuator such that actuation of the handle actuator causes axial movement of the medical tool and the flexible electronics flattening member, to thereby maintain the flexible electronics bundle in a flattened configuration, optionally between the first and second locations.

The medical device may also include a flexible electronics bend prevention member.

One aspect of the disclosure is a method of using a medical device that is configured and sized to be positioned within a subject. The method may include positioning an ultrasound transducer of an intravascular ultrasound catheter within a subject, wherein flexible electronics (e.g., a flexible conductor bundle) that are secured to the ultrasound probe at a first location extend proximally therefrom to a handle assembly; proximally retracting the ultrasound transducer; and applying tension to the flexible electronics at a second location that is proximal to the first location. The second location may be within the handle assembly.

The method may further comprise actuating a handle actuator, wherein actuating the handle actuator causes the proximal retraction of the ultrasound transducer and causes the application of tension to the flexible conductor bundle at the second location within the handle assembly.

Applying tension to the flexible electronics may prevent folding in the flexible conductor bundle distal to the second location.

Applying tension to the flexible electronics may comprise moving a tensioning member proximally within the handle assembly, the tensioning member secured to the flexible conductor bundle at the second location. The tensioning member may be in operative communication with a handle actuator, the method may further comprise actuating the actuator to cause the proximal movement of the tensioning member and the application of tension to the flexible conductor bundle.

The medical device may include flexible electronics that have a twisted configuration in a twisted region along at least a portion of its length.

These and other features and advantages are described in greater detail below.

### BRIEF DESCRIPTION OF THE DRAWINGS

Non-limiting and non-exhaustive examples are described with reference to the following figures.
Figure 1A illustrates an example embodiment of a system that includes steering and a medical device.
Figure 1B illustrates a cross section A-A of the steering and device portion of the medical device of Figure 1A.
Figure 2 illustrates an example system that includes a handle assembly with a plurality of actuators, a steerable sheath and medical tool.
Figures 3Ai-3Aiii illustrate an example steerable shaft with pull wires.
Figures 3Bi and 3Bii illustrate an example steerable shaft with pull wires.
Figures 3Ci and 3Cii illustrate an example steerable shaft with pull wires.
Figures 3Di-3Div illustrate an example steerable shaft with pull wires.
Figure 3E illustrates an example steerable shaft with one or more pull wires circumferentially interwoven into braid wires of the shaft.
Figure 4 illustrates an example portion of an example system that includes a bundle.
Figure 5 illustrates an example proximal end of a medical tool, the tool including a conductor bundle that extends into a proximal connector within which is housed a printed circuit board (PCB).
Figure 6A illustrates a portion of an example medical tool that includes a flexible circuit strip.
Figure 6B illustrates an example proximal portion of a strip.
Figure 6C illustrates a detailed view of an example proximal portion of a strip.
Figure 6D illustrates an end view of an example flex strip.
Figure 6E illustrates an example stack of flex strips.
Figure 6F illustrates an example stack of flex strips and ground and shield strips.
Figure 6G illustrates an example bundle including a tubing material around a stack of strips and shield and ground strips.
Figure 7 illustrates an integrated system of the steerable sheath and medical tool wherein the system is connected to a console via a connector cable.
Figures 8A and 8B illustrate an example handle assembly that can be used with any of the inner and outer elongate bodies or shafts herein.
Figure 9A illustrates a portion of an example inner elongate body, or inner shaft.
Figure 9B illustrates a portion of an example outer elongate body, or outer shaft.
Figure 9C illustrates a portion of an example medical device including the elongate bodies (or shafts) from figures 9A and 9B.
Figure 9D illustrates a section of the device in the deflectable portion from figure 9C.
Figure 10A illustrates a portion of an example handle assembly.
Figure 10B is an exploded view illustrating an example outer elongate body (or outer shaft) movement subassembly.
Figure 10C illustrates a side sectional view of the handle assembly from Figure 10A.
Figure 11 illustrates an example handle assembly that includes rotation indicators for first and second actuators.
Figure 12A illustrates internal components of an example handle assembly that includes a tensioning member, or flattening member.
Figure 12B illustrates internal components of an example handle shell that include one or more guiding features positioned to help stabilize a tensioning member in the handle assembly.
Figure 12C illustrates a side view of an example handle shell.
Figure 12D illustrates (in a side view) internal components of an example handle assembly that includes a tensioning member, or flattening member.
Figure 12E illustrates a side view of an example handle shell.
Figure 12F illustrates (in a side view that is the other side relative to figure 12D) internal components of an example handle assembly that includes a tensioning member, or flattening member.
Figure 13A illustrates an example flexible cable bundle with at least a portion that is twisted relative to a long axis.
Figure 13B illustrates an example flexible cable bundle with at least a portion that is twisted relative to a long axis.
Figure 13C illustrates an example flexible cable bundle with at least a portion that is twisted relative to a long axis.
Figure 13D illustrates an example flexible cable bundle with at least a portion that is twisted relative to a long axis.
Figure 13E illustrates a detail from figure 13D.
Figures 14-18 illustrate example medical devices with navigation interface.
Figures 19-24 illustrate example transducer tips for medical devices.
Figure 25 illustrates an example assembly for a medical device.
Figure 26 illustrates an arrangement of navigation sensors for a medical device.
Figures 27A-D and Figures 28 and 29 illustrate various configurations of pull wires used to deflect a portion of a shaft or sheath of a medical device.

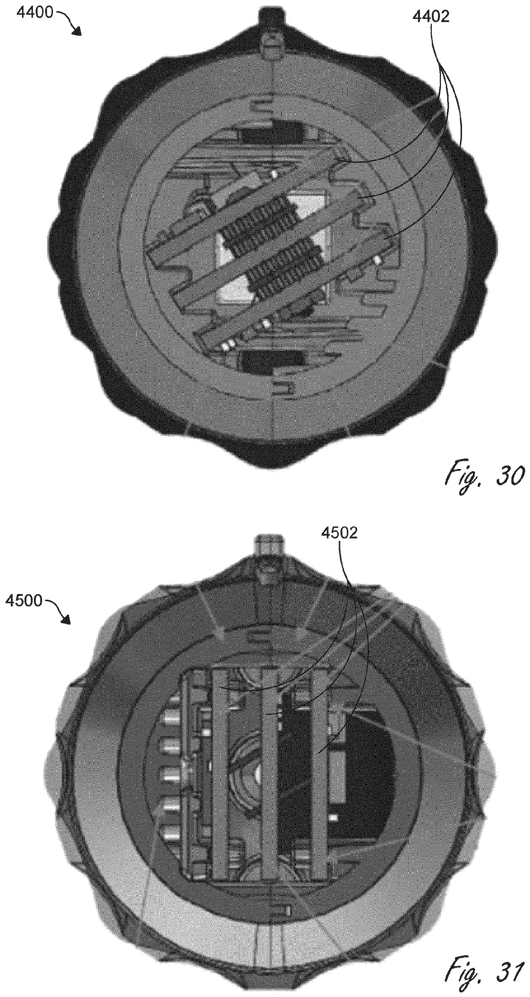

Figures 30-31 illustrate various arrangements of components in a handle assembly of a medical device.
Figures 32A-32B illustrate an example of aspects of a portion of a shaft or sheath of a medical device.
Figures 33A-33B illustrate example deflections and tip lengths of a medical device.
Figure 34 illustrates example deflections and tip lengths of a medical device.
Figure 35 illustrates example dimensions of a medical device.
Figure 36 illustrates example dimensions of a medical device.
Figure 37A illustrates an example folding pattern of flexible strips in a tip of a medical device.
Figure 37B illustrates an example folding pattern of flexible strips in a tip of a medical device.
Figure 38 illustrates an example folding pattern of flexible strips of a medical device.
Figure 39 illustrates an example folding pattern of flexible strips of a medical device.
Figure 40 illustrates an example folding pattern of flexible strips of a medical device.
Figure 41 illustrates an example tip of a medical device with sensors.
Figure 42 illustrates an example diagram of axes of a medical device according to the present disclosure.
Figure 43 illustrates an example diagram of axes of a medical device according to the present disclosure.
Figure 44 illustrates an example medical device configuration.
Figure 45 illustrates an example medical device configuration medical device configuration.
Figure 46 illustrates an example medical device configuration medical device configuration.
Figure 47 illustrates an example medical device configuration medical device configuration according to the present disclosure.
Figure 48 illustrates an example medical device configuration medical device configuration according to the present disclosure.
Figure 49 illustrates an example medical device configuration medical device configuration according to the present disclosure.
Figure 50 illustrates an example medical device configuration medical device configuration.
Figure 51 illustrates an example medical device configuration medical device configuration according to the present disclosure.
Figure 52 illustrates an example medical device configuration medical device configuration.
Figure 53 illustrates an example medical device configuration medical device configuration according to the present disclosure.
Figure 54 illustrates an example medical device configuration medical device configuration.
Figure 55 illustrates an example medical device configuration medical device configuration according to the present disclosure.
Figure 56 illustrates an example medical device configuration medical device configuration according to the present disclosure.
The accompanying drawings show examples of the disclosure. It is to be understood that the examples shown in the drawings and/or discussed herein are non-exclusive and that there are other examples of how the disclosure may be practiced.

### DETAILED DESCRIPTION

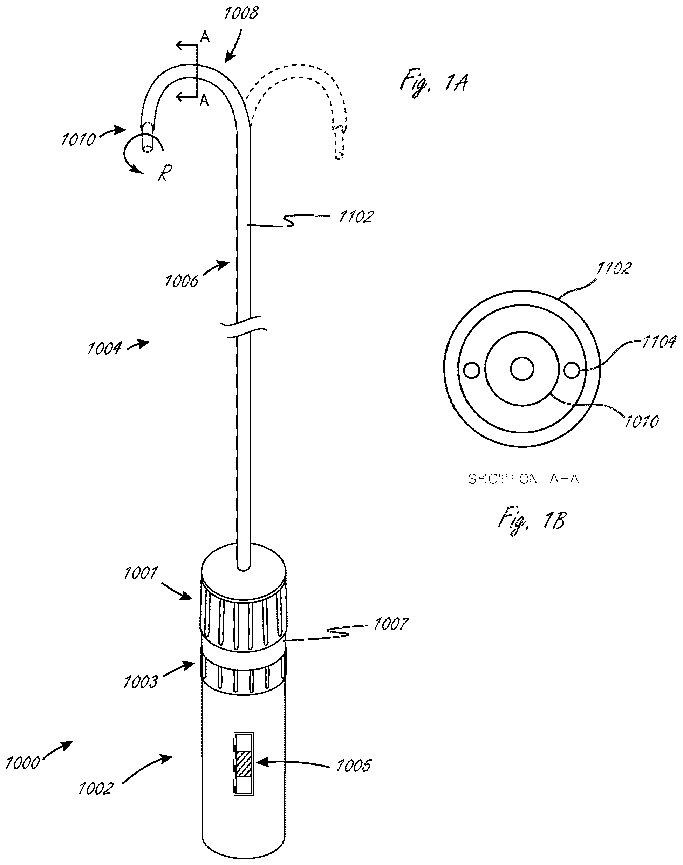

Figure 1A illustrates an example embodiment of a system that integrates steering and a medical device. System 1000 includes handle assembly 1002 and steering and medical device portion 1004. Steering and medical device portion 1004 includes a proximal portion 1006 and steerable portion 1008. The system is adapted so that handle assembly 1002 can be actuated to cause steering of the steerable portion 1008, and optionally can be further actuated to cause movement of medical device 1010 relative to steering and medical device portion 1004. In this example embodiment, handle assembly 1002 includes first actuator 1001, second actuator 1003, and third actuator 1005. First actuator 1001 is adapted to be actuated (in this example rotated) relative to handle body 1007 to cause the steering of steerable portion 1008, and specifically steering outer sheath 1102. Steerable portion 1008 in this embodiment can be steered, or bent, into the configuration shown in Figure 1A in solid lines, and can also be steered into the configuration shown in dashed lines, or anywhere in between, and in some embodiments the opposite steering function is limited to simply straightening the shaft from an initial bent configuration, such as the solid line bent configuration in Figure 1A. The term "steer" in this disclosure means to deflect or bend, optionally via actuation of at least one pull wire, but in some instances the term can include shaft rotation (torqueing) and axial movement. The term "pull wire" herein refers to any element that may transmit a tensile force from the proximal end of the device to the distal end region. Pull wires may be comprised of metal wire such as stainless steel or nickel titanium, either solid or stranded/braided, or it may be comprised of a polymer such as aramid fiber (Kevlar^{®}), polyethylene, ptfe, eptfe, etc., preferably stranded/braided, but also in monofilament form. In a preferred embodiment, the pull wire is constructed from an aramid fiber bundle having four 50 denier multifilament (approximately 25 filaments) threads braided together at a high picks per inch. The wire cross-sectional diameter is typically in the .005"-.012" range, more preferably .008"-.010", although braided or stranded wire may flatten or ovalize in the device lumen. The preferred construction embodiments are believed to provide optimized strength and wear resistance for the size necessary to keep the shaft diameters to a minimum. Optional second actuator 1003 is adapted to be actuated relative to handle body 1007 (in this example rotated) to cause rotation of medical tool 1010 relative to shaft 1102 (labeled as rotation movement "R"), and optional actuator 1005 is adapted to be actuated relative to handle body 1007 (in this example axially) to cause axial (distal-proximal) movement of medical device 1010 relative the outer sheath 1102. Proximal portion 1006 is not configured to bend significantly when steerable portion 1008 is steered (bent/deflected), although the proximal portion may flex and bend to conform to the anatomy within which it is used. In many embodiments, this is accomplished by constructing the steerable portion 1008 from a softer or less rigid material and/or composite construction than the proximal portion 1006.

The embodiment shown in Figure 1A is an example of an apparatus that includes an integrated handle assembly that is in operable communication with both a steerable outer shaft and an inner medical tool. The handle assembly is integrated in that it is assembled and constructed to be in operable communication with the outer shaft and the inner medical tool prior to packaging and use. "Integrated" as that term is used in the context of an integrated handle assembly refers to a handle assembly in which at least one part of the handle assembly has to be broken or taken apart before the medical tool can be removed from within the outer shaft.

Figure 1B illustrates an example cross section A-A (shown in Fig. 1A) of the steering and device portion 1004, and specifically in the steerable portion 1008. In this embodiment medical device 1010 is sized and configured to be disposed within a steerable sheath. The steerable sheath includes an outer shaft 1102 and a set of pull wires 1104, which are axially fixed in a distal region of steerable portion 1008.

The medical tool in Figures 1A and 1B can be, for example, any medical tool herein, such as an ultrasound tool. When "ultrasound probe" is used herein, it generally refers to an elongate tool that includes at least one ultrasound transducer and one or more conductive elements that electrically connect the at least one ultrasound transducer to a proximal region of the elongate tool. A proximal region of the ultrasound probe includes, or is modified to include, at least one proximal contact, which is in electrical communication with the at least one ultrasound transducer, and which can be put into electrical communication with, optionally via attachment to, an electrical contact on another device, cable, or connector.

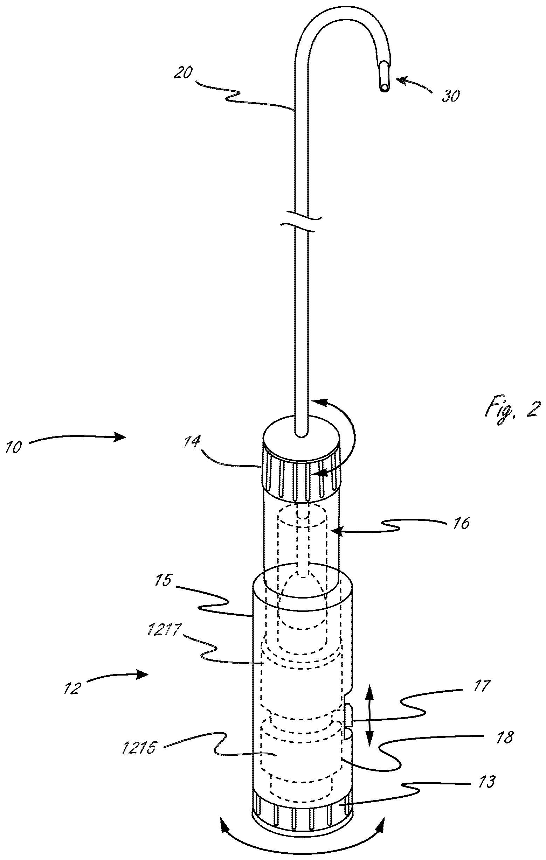

Figure 2 illustrates an example system 10 that is adapted to function similarly to the system in figures 1A and 1B, and also illustrates example internal components of handle assembly 12 (internal components shown as dashed lines). Handle assembly 12 is integrated and in operable communication with outer steerable shaft 20 and medical tool 30. Handle assembly 12 includes actuator 14 that is adapted to, when actuated relative to handle body 15, cause steering of steerable shaft 20. Actuator 14 is in operable communication with steerable shaft 20 via steering control 16 disposed in handle assembly 12. Medical tool 30 includes a proximal portion 18 disposed within and incorporated into handle assembly 12. Actuator 13 is in operable communication with medical tool 30, and actuation of actuator 13 (in this example rotation) relative to handle body 15, causes rotation of medical tool 30 relative to outer shaft 20 via rotation control 1215. Optional third actuator 17 is also in operable communication with medical tool 30, and is adapted to be actuated, in this embodiment, axially (relative to handle body 15), to cause axial movement of medical tool 30 relative to outer steerable shaft 20 via axial control 1217.

The medical tool in Figure 2 can be, for example, any medical tool herein, such as an ultrasound tool.

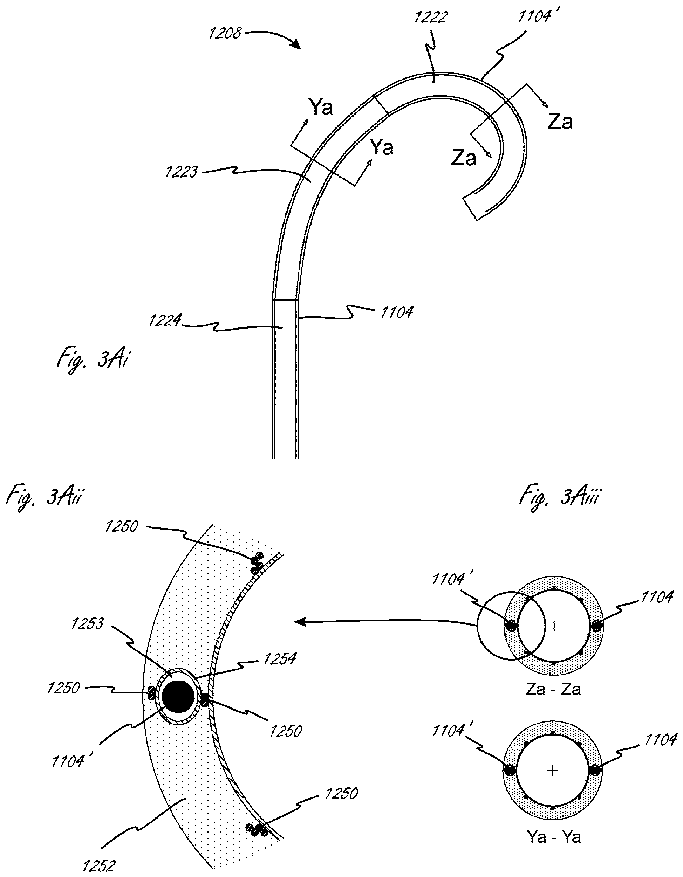

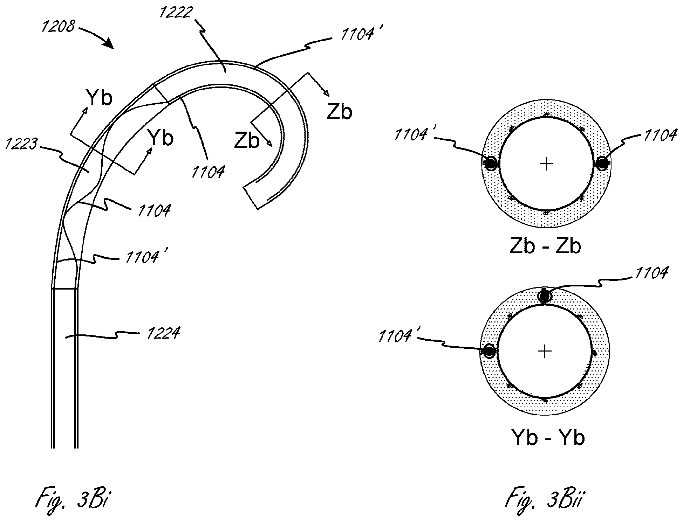

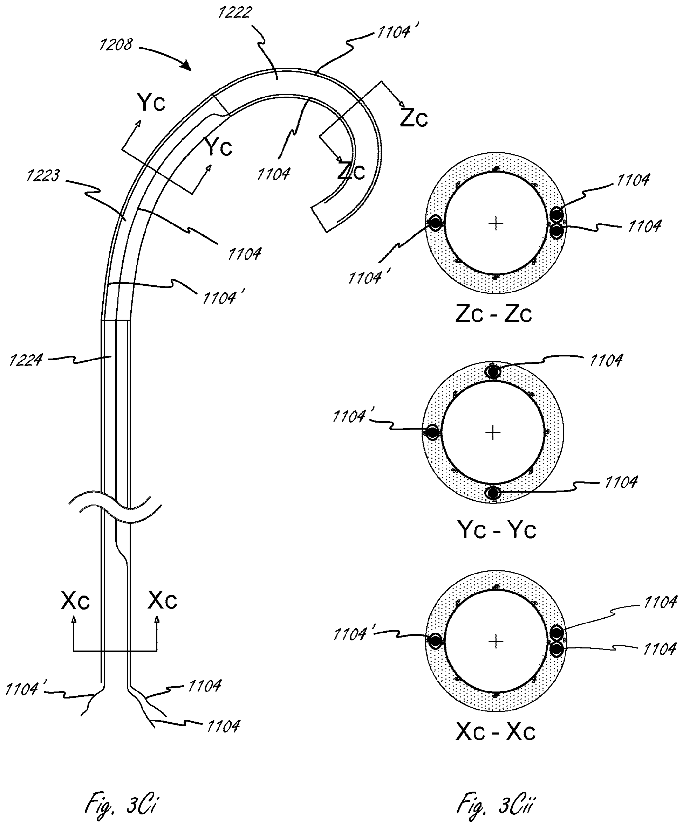

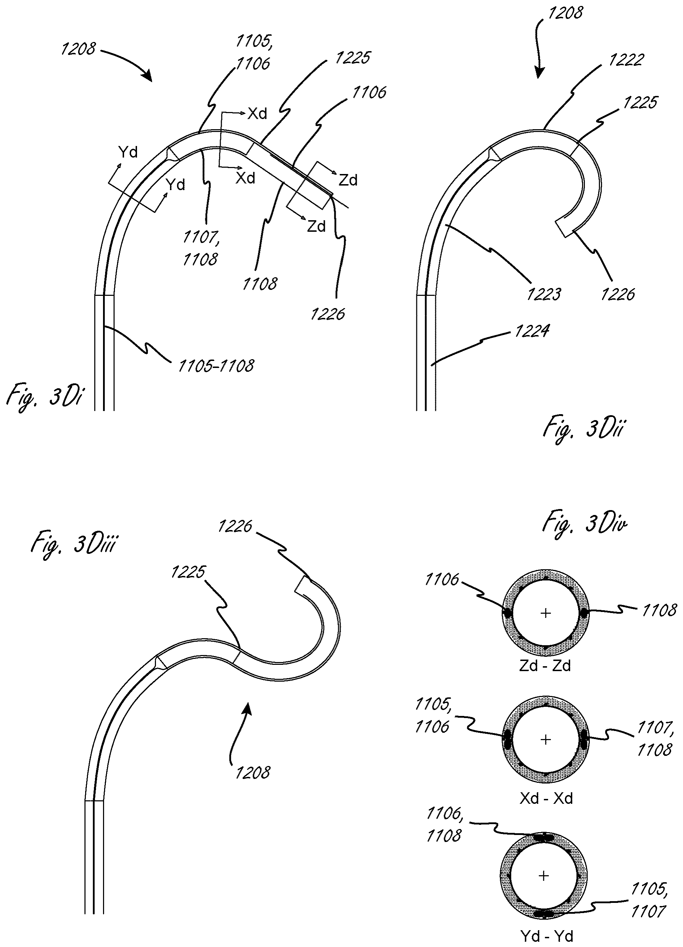

Figures 3A-3E represent example embodiments of a distal region of the sheath portion 1208 of steerable sheath 1202 in system 1200. For simplicity, the illustrated cross-sections show only the outer sheath 1208 and not the inner tool 1212. The outer sheath 1208 preferably has a composite construction to improve torque transmission applied to the outside of the shaft from the proximal end, or to resist torque forces applied to it from within the shaft, such as from tool 1212. As illustrated in Figure 3Ai-iii, in order to form the composite, multiple braid elements 1250, preferably formed from metal wire (round, pairs of round, or ribbon shaped) and/or multiple fibers (e.g., aramid or nylon), may be braided directly over a thin wall (e.g., .0010" ± .0005") lubricious liner tube 1251, such as a PTFE or FEP material. A thermoplastic polymer 1252 (such as Pebax in a range of durometers from 25D-72D, or nylon, or other common catheter materials) may be laminated with heat using heat shrink tubing (such as FEP) to reflow the polymer over the braid elements 1250 and liner tube 1251 to form a uniform member. The thermoplastic polymer 1252 may also have radiopaque compounds that include materials such as bismuth, barium sulfate, or tungsten in order that the tip of the sheath be visible to the user under fluoroscopy.

In the embodiment of Figure 3Ai-iii, the pull wire 1104 is preferably parallel to a central axis in the steerable (deflectable) portion 1222 of the sheath and also preferably provided in a lumen 1253 created within the wall of the steerable sheath 1208. This lumen may be created during the thermoplastic polymer tubing extrusion process or during a shaft heat lamination fusing process with the aid of a removable mandrel. The pull wire lumen 1253 may further be created by incorporating a pull wire tube 1254, preferably temporarily supported by a removable mandrel, within the wall. The removable mandrel may also be placed alongside the pull line 1104 or 1104' during the fusing process, resulting in a somewhat ovalized lumen 1253 within which a fiber pull wire may be allowed to flatten into, allowing space for free movement of the pull wire. The tube 1254 may include PTFE, FEP, polyimide, or another material which maintains its wall integrity during a heat lamination process up to approximately 500 °F. The tube is preferably surrounded and supported by the thermoplastic polymer 1252 which is preferably heat laminated against the tube.

In another embodiment, the pull wire lumen, preferably comprising the pull wire tube, is incorporated within the weave of the braid elements 1250. For example, braid elements 1250 running in one direction would pass under the pull wire lumen, while those running in the opposite direction would pass over the pull wire lumen. The braid reinforcement provides a more dimensionally stable lumen during catheter manipulations and also helps assure the straightness of the lumen as needed. Proximal to the steerable portion, the pull wire may continue proximally parallel to the central axis on the same side of the outer sheath 1208, such as is illustrated in Figure 3Ai-iii. In this embodiment and others that follow, an additional pull wire 1104' within an additional pull wire lumen routed within the wall of sheath 1208, up through the steerable portion 1222, may be required to straighten the steerable portion of the device. This straightening pull wire 1104' is preferably routed within steerable portion 1222 on the side opposite from the pull wire(s) 1104 used for steering (deflection) in the steerable portion 1222. In another embodiment, not shown, two lumens and two straightening pull wires 1104' could be used, essentially mirroring the paired 1104 pull wire configuration. These straightening wires could also be constructed to allow deflection in the opposite direction by tensioning a greater distance (beyond just straightening) within the handle.

During use, a portion 1223 of the distal catheter just proximal to the steerable (deflectable) portion 1222 may be forced to conform to a curve based on the constraints of the anatomy in which it is used. For a specific embodiment where the device is advanced into the heart chambers from a groin access, the portion 1223 forced into a curve is expected to range from 5 to 25 cm in length. During rotation of the sheath shaft 1208 from the proximal end, torque is transmitted through this distal curved region 1223 to the catheter tip. A non-uniform cross section and/or tension of the device in this region 1223 may induce a tendency for the shaft to build up and suddenly release torque, causing a "whip" or sudden jerk in rotation as it is torqued. To minimize the potential for whip, it is optional to distribute the pull wire tension and construction material around the surface of the curved region 1223. In one embodiment, such as is illustrated in Figure 3Bi-iii, the pull wire 1104 may spiral around the central axis of the sheath in at least the curved region 1223 proximal to portion 1222. The pull wire of this embodiment may make a full circumferential wrap over approximately 10 cm of length, with this value ranging 5-15 cm. The spiral may only need to be present in the curved region 1223, continuing straight proximally thereafter through proximal portion 1224 (similar to 1006), which may minimize the friction in the pull wire lumen and the associated pull wire force required to steer (deflect) the steerable portion 1222. The spiral may also make a minimum of one turn before continuing straight, or spiral the full length of the shaft. In another embodiment to minimize whip, it may only be necessary to distribute the pull wire tension to opposite sides of the shaft. As illustrated in Figure 3Ci-ii, deflection of the steerable section 1222 is accomplished with two parallel pull wires 1104 positioned adjacent one another on the same side of the sheath 1208. In the curved region 1223 and proximal portion 1224 (similar to 1006) proximal to the steerable section 1222, the pull wires are routed to opposite sides of the shaft, each 90° from the position in the steerable section 1222, to distribute the tension more evenly. While it is preferable to actuate the two parallel pull wires at the same time with equal force with the handle actuator, in other embodiments, a differential in force could be applied to steer the tip to one side or the other of the plane formed when the two are actuated with equal force. In other embodiments, any plurality of pull wires could be routed in the same configuration as illustrated in Figures 3B or Figure 3C, with the multiple proximal pull wires distributed uniformly around the shaft circumference. Also, as illustrated in Figure 3Ci-ii, the pull wires 1104 may be routed proximally along the opposite sides of the shaft for most of the shaft proximal portion 1124 length, but preferably brought back together adjacent one another near the proximal end portion of the shaft to allow the wires to exit the same side of the proximal shaft together to facilitate them being secured together to a handle component for simultaneous actuation tension.

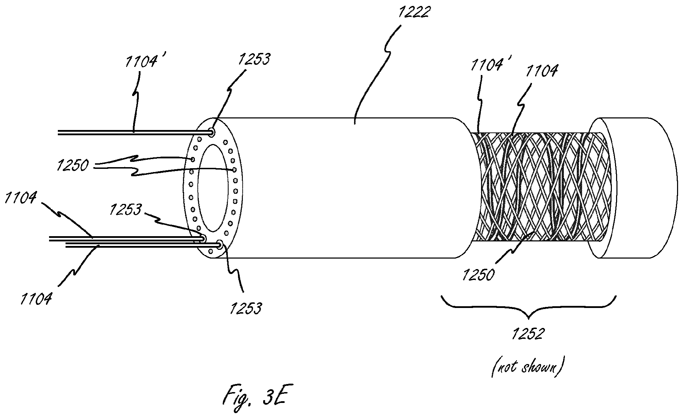

Figures 3Di-iv illustrate another embodiment of the distal region of catheter with construction similar to that previously described, but instead configured to provide a distal steerable portion 1222 which can be deflected into two different directions. As illustrated, a two pairs of pull wires 1105/1107 and 1106/1108 are along the proximal shaft region 1224 and curved region 1223. This is similar to Figure 3Ai-iii, except that the wires are paired on each side of the shaft. The routing could also be spiraled as in Figure 3Bi-ii, or other configurations discussed. Within distal steerable portion 1222, the wires are routed 90° from the proximal portions, although other angles are contemplated. At a junction 1225 within 1222 one or more of the pull wires (e.g., 1105 and 1107) may be terminated and anchored to the shaft, with the remaining pull wires (e.g., 1106 and 1108) continuing to a more distal tip location 1226 where they are anchored. This configuration allows independent actuation of pull wires terminated at 1225 and 1226 such that different shapes may be created during actuation. Figure 3Dii shows both lines 1107 and 1108 tensioned to create a variable curve in the same direction. Figure 3Diii shows lines 1107 and 1106 tensioned to create an "S" curve. Other configurations are also possible.

The pull wires (such as 1104 and 1104') may be terminated at their distal end in a manner that reliably affixes them to the wall of the distal steerable shaft portion 1222, such that they do not break or pull free under repeated applications of tension. In a preferred embodiment, shown in Figure 3E, the pull wires 1104 and 1104', upon exiting the distal pull wire lumen 1253, are circumferentially interwoven into the braid wires 1250 of the distal shaft 1222 (shown without the thermoplastic polymer 1252). One or more of the pull wires 1104 or 1104' may also be additionally or instead wrapped and/or tied around the outside of the braid wires 1250 for additional securing. The braid wires 1250 may be then trimmed distal to the securing point, with the interwoven and/or wrapped pull wires preventing the braid wires from expanding and/or unraveling. Additional adhesives such as UV cured or cyanoacrylates may also be used to secure the pull wires to the braid wires. The weave and/or wrap of the pull wires and braid wires is then laminated with a thermoplastic polymer which melts within the space around the wires and cools to secure them in place. The thermoplastic polymer may also have radiopaque compounds that include materials such as bismuth, barium sulfate, or tungsten in order that the tip of the sheath be visible to the user under fluoroscopy.

In additional embodiments, the tool 1212 may also or alternatively be constructed with one or more pull wires to deflect the tip in a manner similar to any of the previous embodiments described for the outer sheath 1208. In addition to routing the pull wires within the wall of the tubular member of the tool 1212, the pull wires could be routed next to the conductors inside the lumen of the tubular element 1212. Actuation of the pull wires could be from an actuator located in the proximal handle 1206. The distal shaft of tool 1212 may also be formed into a particular shape (e.g., an arc) such that it bends into the shape as it exits the tip of the steerable portion 1222 of outer sheath 1208. The stiffness of the distal shaft of tool 1212 is such that it does not substantially deform outer sheath 1208 while inside, but upon exiting is allowed to bend. The shape may be set by any one or combination of the following means: heat setting the polymeric material, using a moveable or fixed shaped stylet within the inner lumen of shaft 1212 or within a lumen within the wall of shaft 1212. Such a stylet could be round, oval, or rectangular in cross section, and be formed of stainless steel, nitinol, or a rigid polymer such as PEEK, Vestamid, or similar. The outer steerable sheath could alternatively be made to bend with a similar method as above, with or without additional pull wire deflection, and with or without additional shape or deflection of the distal portion of tool shaft 1212.

One aspect of the disclosure includes methods of disassociating at least a portion of the system from other components, optionally as part of a reposing process. In some embodiments the medical tool includes one or more electrical contacts that are coupled to other electrical contacts, which are in electrical communication with an energy console, and examples of consoles are known in the ultrasound art.

In an embodiment, the pull wires described in Figures 3Ai-3E may exist in a symmetric design and may be bifurcated and change relative position to each other in various sections of a respective shaft. By controlling a stiffness in a shaft and a positioning of the pull lines (e.g., moving a bifurcation point more distally), off-axis curl may be reduced, while providing more precise control of a deflection of the distal end of the shaft. An associated shaft may comprise a plurality of bobbins (e.g., sixteen (16) bobbins), which may be tuned for increased torque response. An associated shaft may comprise a plurality (e.g., four (4)) of pull lines) for balanced braid wire, which may decrease deflection orientation changes due to annealing and sterilization. An associated shaft may comprise a more optimized non-deflecting tip length, as described herein. As a further example, an associated shaft may comprise a flaring inside diameter of a tip to not bind on bundle, just proximate to post.

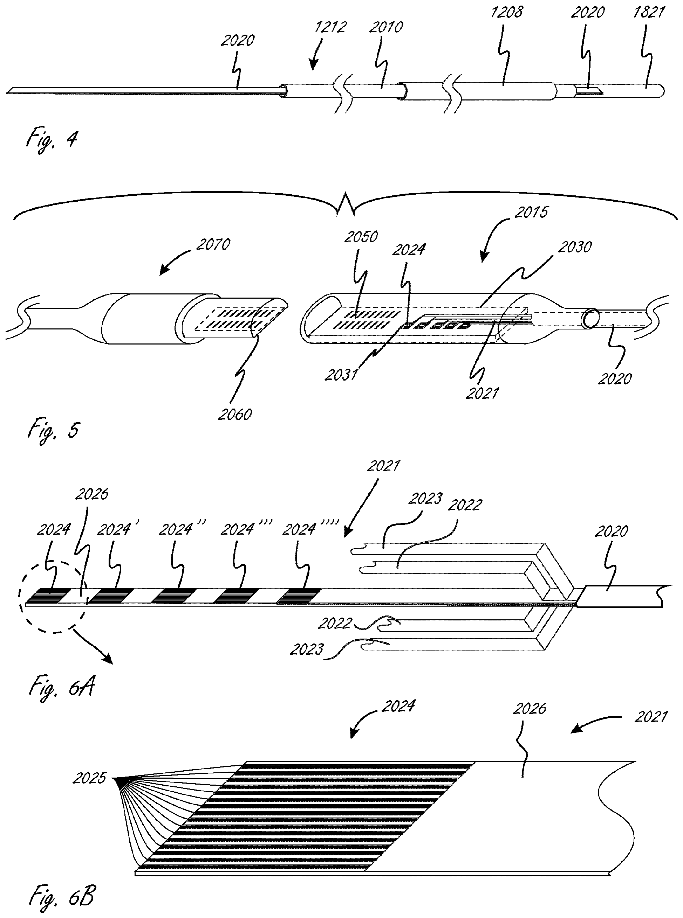

Figure 4 illustrates a portion of an example medical tool, such as an ultrasound probe, that can be electrically coupled directly or indirectly to an energy console, such as an ultrasound console.

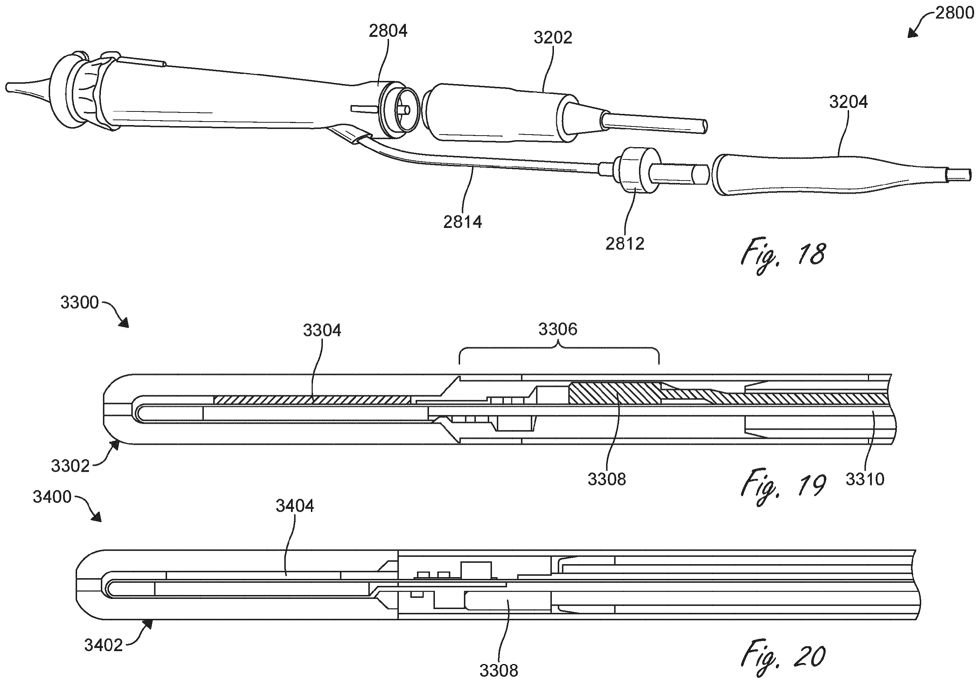

Reposing the device can involve disconnection of one or more proximal electrical contacts and moving the tool portion distally out of the distal end of the sheath portion. In this embodiment tool portion 1212 comprises at least a tool outer sheath or member 2010, distal working end 1821 (which can include at least one ultrasound transducer), and conductor bundle 2020. The conductor bundle 2020 extends from the distal working end 1821, through the tool outer member 2010 to a proximal connector (the connector and handle mechanism are not shown in Fig. 18 for clarity). In some embodiments the medical tool is used for ultrasound imaging, optionally where the distal working end 1821 comprises a two-dimensional (2D) array of piezo electric components mounted on an ASIC (application specific integrated circuit).

Figure 5 illustrates a merely example proximal end of a medical device (the medical device is shown on the right), and in this embodiment the medical device is an ultrasound probe. The proximal end 2015 of the medical device is adapted to be electrically coupled to connector cable 270, which is directly or adapted to be indirectly electrically coupled to an energy console, such as an ultrasound energy console. As illustrated in Figure 5, flexible conductor bundle 2020 extends from a distal region of the medical tool (distal region not shown) into a proximal connector 2015 within which is housed a rigid or flexible printed circuit board ("PCB") 2030. The connector bundle 2020 includes a plurality of contacts 2024 (examples of which are described below) that are attached to PCB board contacts 2031. Each individual trace from each contact 2031 is linked to individual exposed contacts 2050 on another portion, optionally more proximal, of the PCB. The individual PCB traces may also pass through other useful circuitry on the PCB. The exposed contacts 2050 are configured for a mechanical mating for electrical conduction to similar contacts 2060 on mating connector cable 2070, similar in concept to the proximal tool connector 1990 described previously, which links the tool 1204 to a user-interface console. Proximal connector 2015 can be incorporated into any of the systems, handles, steerable sheaths, medical tools, etc., herein.

"Conductor bundle" as that term is used herein may be interchangeably used with "flexible conductor bundle" unless indicated to the contrary herein.

Figures 6A and 6B illustrate an example conductor strip (also referred to herein as a flexible circuit strip) 2021 that can be included in any of the conductor bundles herein. The embodiment in Figures 6A and 6B is an example of a conductor strip that can be included in bundle 2020 from Figures 4 and 5. The embodiment in Figures 6A and 6B can be incorporated into any other system herein.

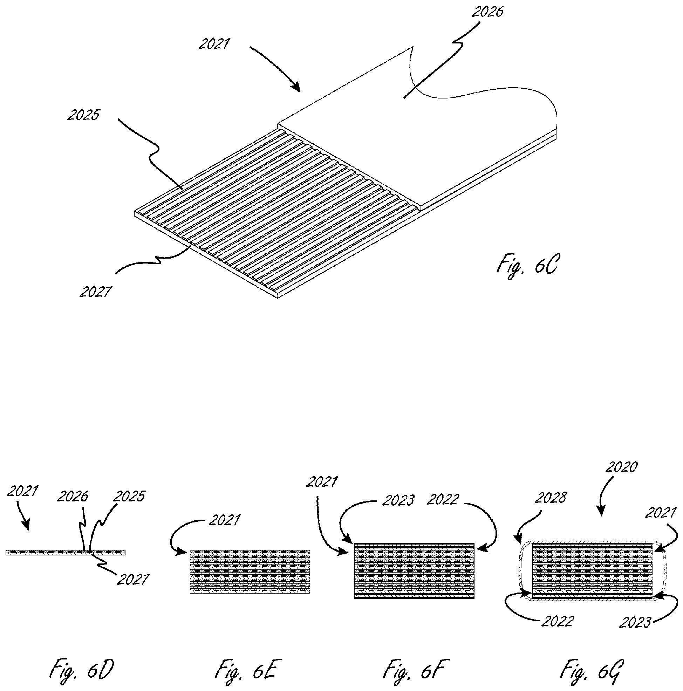

As shown in Figures 6A, 6B and 6G, conductor bundle 2020 comprises a plurality of flex circuit strips, including multi-trace strips 2021, as well as conductive strips for grounding 2022 and shielding 2023 (only a portion of which are shown). Each multi-trace strip comprises a plurality of conductive traces 2025, which can be seen clearly in Figures 6B, 6C and 6D. The number traces 2025 in Figures 6D-G is twelve, and the number of traces in Figures 6A-6C is sixteen, and they are both example as to the number of traces 2025 that can be used. Each strip 2021 can be approximately .072" wide and .0022" thick, and can optionally comprise sixteen .0022" wide x about.0007" thick conductive (e.g., copper) traces, each spaced approximately .0022" apart. The traces are disposed on an insulating substrate layer 2027, such as a polyimide substrate, and the traces can be at least partly covered by a cover layer 2026, such as a photoimageable film cover ("PIC") layer or other dry film solder mask (DFSM) or other similar material. The cover layer generally extends along most of the bundle, except at discrete locations in proximal and distal regions for electrical coupling. In other embodiments, the strip 2021 is approximately .055" wide and comprises twelve conductive traces (see figures 6D-G). In other embodiments, the strip 2021 is approximately .037" wide and comprises eight copper conductive traces. The outer strips 2022 and 2023 used for grounding and shielding may have a similar construction and dimension except they can comprise a single full width strip of copper. As optimized for a 2D piezo array, a stack of approximately seven 16-trace strips 2021 would be required (or nine 12-trace, or fourteen 8-trace), along with one each of strips 2022 and 2023 on each side of the stack of multi-trace strips. Figure 6E illustrates a portion of an example bundle 2020 with nine strips 2021 stacked together. Figure 6F illustrates a portion of the bundle that includes nine strips 2021 stacked, as well as ground strip 2022 and shield strip 2023 (only those on top are labeled). The complete bundle may optionally be held together with a, for example without limitation, about.001" wall thickness shrink tube, such as the tubing 2028 in Figure 6G. The flex circuit dimensions and number of traces discussed above are for a particular configuration of a piezo-electric array (and/or an ASIC controller thereof) and may be varied depending on how the number and size of array elements are optimized for the particular application.

The proximal end of each flex circuit strip has the conductive material (e.g., gold-plated copper) exposed over a length of approximately, for example, 3mm through removal of the cover layer 2026 at location 2024. Location 2024, and other exposed locations described herein, is generally referred to as a "contact." It is understood that when used in this context, the contact actually includes a plurality of separated conductive traces (such as shown in region location), each of which is adapted to be in electrical communication with its own corresponding conductive element. "Contact" is therefore not limited to mean only a single electrical connection between two conductive elements. While Figure 6A shows a plurality of exposed regions 2024, the embodiment in Figure 6A will first be described herein as if there is only one exposed region (i.e., region 2024 at the proximal end). The strip 2021 can be made to create an electrical connection to matching exposed contacts 2031, shown in Figures 6A-C, for conductive traces on the PCB 2030. In some embodiments, sixteen individual traces, sized and spaced to match sixteen traces in the multi-trace strip 2021, would be provided within a given contact 2031. An ACF (anisotropic conductive film), soldering, conductive adhesive, mechanical connection, or any combination of these may be used to achieve a suitable electrical connection (electrical coupling) between the strip traces and the PCB contacts.

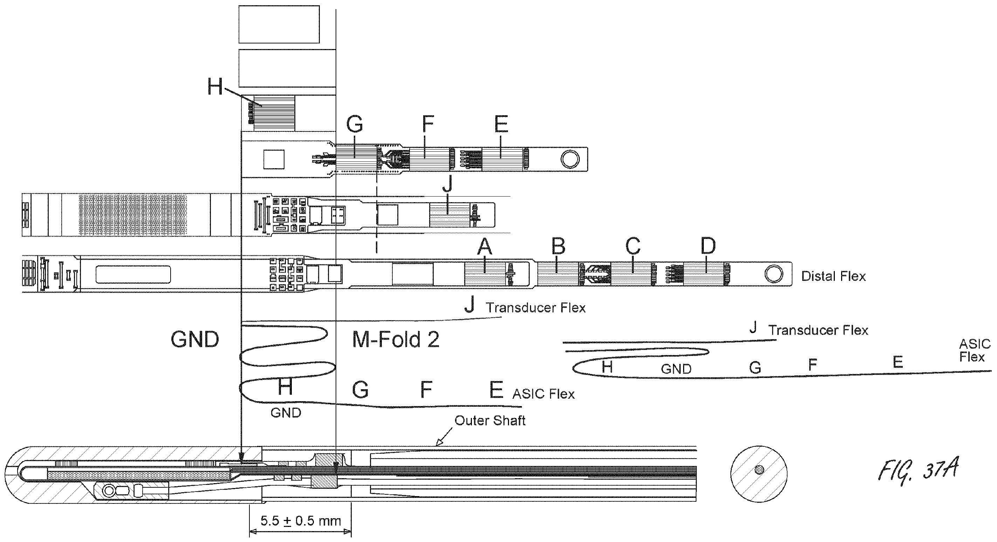

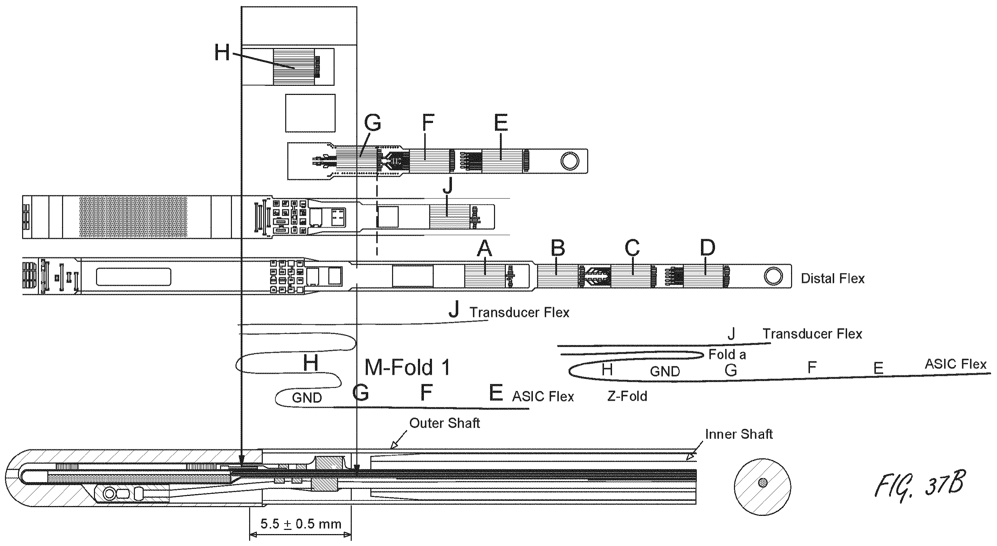

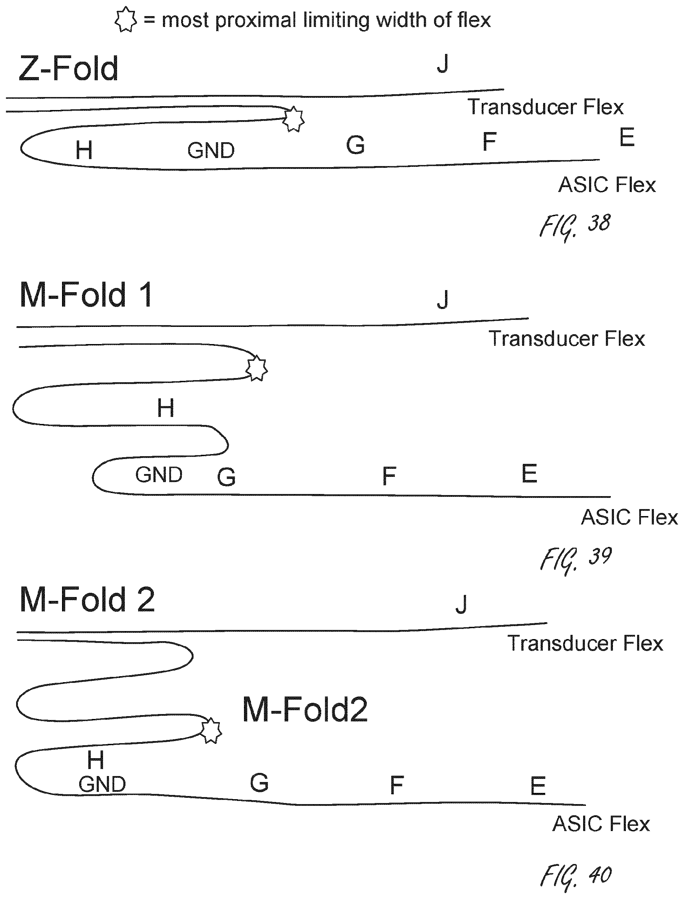

The flexible strips shown in Figures 6A-6G may comprise a variety of configurations. A modified M-fold configuration that pulls a wider portion of the application-specific integrated circuit (ASIC) flex further distal may allow for more distal positioning of the inner shaft 132. A Z-fold configuration may comprise two bends. As an illustrative example, a stacked configuration of the flexible circuit board may be used. A Z-fold is illustrated in Fig. 38, for example, and shows the stacked components J, H, and G as referenced in Fig. 37. A first version of an M-fold configuration may allow for a short length associated with a tip, as shown in Fig. 39. The modified M-fold configuration may allow for a shorter length associated with the tip than the other two configurations described, as shown in Fig. 40.

The modified M-fold configuration may comprise a series of folds in the flex such that a proximal wide portion of the flex is brought as far distal as possible. The optimal number of fold points is 4, above which the stack is too thick to fit the outer shaft. The wide portion of interest is just distal to pad G, which is too wide for the inner diameter of the inner shaft to advance over. Some fold locations, particularly those that include a fold of the long flex (e.g., connecting to pad H), may need to be reinforced to prevent the fold from being too tight that the cover layer and/or copper traces are damaged. Reinforcement over the fold location can be achieved with a variety of materials, such as adhesive, tape, or thin wall heat shrink tubing (e.g., PET), so as to limit the bend radius of the flex to an acceptable level.

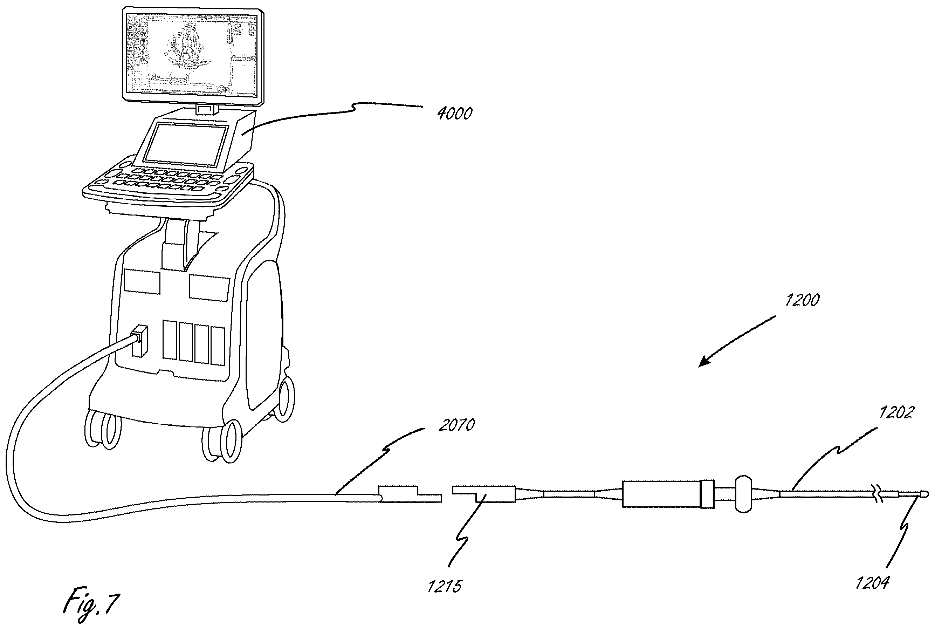

Figure 7 illustrates the integrated system 1200 of the steerable sheath 1202 and medical tool 1204 wherein the system 1200 is connected to console 4000 via the connector cable 2070. As previously described, such as for Figure 5, the tool 1204 comprises a proximal connector 2015 which forms a mating connection to cable 2070. As previously described, it is desirable to repose (e.g., reprocess and reuse) the system 1200. It is further desirable to ensure that the system is reposed only by the original manufacturer and not an unaffiliated third-party, and to ensure the device is only reused a specified number of times. To control the reposing process, a crypto-authentication chip (crypto-chip) is incorporated into the tool 1204, preferably on the PCB 2030, although other locations, such as within the steerable handle 1206, or within the tip 3000, are contemplated. The crypto-chip is programmable only by the original manufacturer who controls the authentication keys. The console 4000 to which the system 1200 is connected has a Trusted Platform Module (TPM) which also has the authentication keys. During use of the system 1200, the console 4000 is able to authenticate the system 1200 via the crypto-chip and as desired may read and write information to the chip (e.g., via an EEPROM feature). In any of the scenarios discussed, RFID chips, preferably encrypted, may be used to read and transmit data between the console, connector, and the device.

As used herein, "cleaning" can refer to any type of cleaning, such as without limitation: cleaning an interior of an outer shaft using a flushing system of cleaner and/or disinfectant and optionally mechanical scrubbing with small brushes; mechanical cleaning (e.g., wipes, brushes) an outer portion of an outer shaft and/or outer portion of a medical device shaft (e.g., ultrasound probe) with a cleaner/disinfectant, and optionally submerging the shaft in an ultrasound bath of cleaner/disinfectant for a specified period of time; and optical cleaning methods such as comprising using UV light. "Cleaning" as used here does not refer to a specific cleaning process, but rather refers to the general idea of cleaning an object.

The disclosure herein also includes methods of assembling or reassembling any of the subassemblies or assemblies herein, including any of the subassemblies within any of the handle assemblies herein. For example without limitation, the disclosure here includes methods of spooling one or more pull wires over a bearing surface in a spindle support and then around the spindle.

The methods herein also include manufacturing or constructing any of the individual components of any of the subassemblies or assemblies herein. For example, the disclosure includes methods of manufacturing handle shell components that have particular configurations (e.g., guides, walls, etc.) that can accommodate the internal parts that allow the assemblies or subassemblies herein to function as intended.

Regardless of the reference number with which they are labeled, any of the handle assemblies, medical tools, steerable sheaths, and electrical connections herein can be used together in a system in any combination with each other.

Any of the technology, including ultrasound and steering technology, in any of the following U.S. patent references may be incorporated into any of the medical tools, devices, systems, or methods of use thereof herein, the disclosures of which are incorporated by reference herein: 6100626, 6537217, 6559389, 7257051, 7297118, 7331927, 7338450, 7451650, 7451650, 7527591, 7527592, 7569015, 7621028, 7731516, 7740584, 7766833, 7783339, 7791252, 7791252, 7819802, 7824335, 7966058, 8057397, 8096951, 8207652, 8207652, 8213693, 8364242, 8428690, 8451155, 8527032, 8659212, 8721553, 8727993, 8742646, 8742646, 8776335, 8790262, 8933613, 8978216, 8989842, 9055883, 9439625, 9575165, 9639056, and 20080287783.

Any suitable disclosure above can be incorporated into any of the embodiments below. For example, aspects of devices, systems, and methods of manufacture and use are incorporated herein and can be incorporated into any of the embodiments below unless specifically indicated to the contrary.

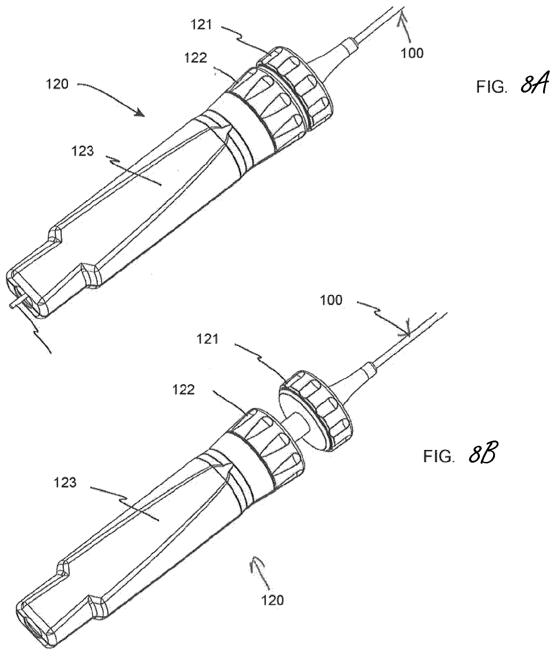

Figures 8A and 8B illustrate a merely example handle assembly that may be in operable communication with outer shaft 131 and inner shaft 132. In this example implementation, handle assembly 120 includes handle body 123 that has an outer surface that can be gripped by a user, first actuator 121, and second actuator 122. Actuator 121 can be in operable communication with outer shaft 131, and actuator 122 can be in operable communication with inner shaft 132. Actuator 121 is adapted to be both rotated and moved axially relative to handle body 123 (and relative to second actuator 122). This allows actuator 121 to cause axial movement of the medical tool 103 and rotation of the medical tool 103 relative to a distal end of inner shaft 132. Second actuator 122 is adapted to be actuated (e.g., rotated in this embodiment) relative to handle body 123 to cause deflection of the inner shaft 132. For example, the handle assembly can have internal components that interface with proximal ends of pull wires such that actuation of actuator 122 tensions one or more pull wires to cause deflection of the inner shaft. In this embodiment actuator 121 is distal to actuator 122, but in other designs their relative positions could be reversed. Figure 8B shows handle assembly 120 after actuator 121 has been advanced distally relative to its position in figure 8A. This distal advancement causes outer shaft 131 to be advanced distally, and thus causes the medical tool to be advanced distally. Actuator 121 can similarly be retracted proximally relative to its position in figure 8B. Tensioning members described further below are referenced with respect to actuator 121 being retracted proximally.

In other designs, actuator 121 could be in operable communication with an inner shaft and actuator 122 may be in operable communication with an outer shaft.

As described herein, the outer shaft can be moved axially relative to the inner deflectable shaft. The outer shaft may be constructed with sections of materials that vary in stiffness (e.g., durometer) along the length of at least a portion of the outer shaft. For example, a first portion that is distal to a second portion can have a lower durometer than the second portion. Because the outer shaft can be moved axially relative to the deflectable inner shaft, and because the stiffness of the outer shaft can vary along its length, the deflection, including the degree (or amount), of the overall device can be selectively controlled by controlling the axial position of the outer shaft (relative to the inner shaft). Axial movement of the outer shaft can thus selectively control deflection of the device. For example, a user (e.g., physician) can change or control where the bend occurs along the length of the device (measured from the distal end) by axially moving the outer shaft relative to the inner shaft. Additionally, for example, sections of varying stiffness in the outer shaft can allow for more or less deflection depending on the relative position of the outer shaft relative to the deflectable inner shaft. For example, deflecting the inner shaft at a region where the outer shaft has a relatively higher stiffness can result in less deflection than when the inner shaft is deflected at a region where the outer shaft has less stiffness. Although reference is made to distinctions in characteristics and control of the inner and outer shafts, such control of the inner shaft and outer shaft may be reversed, wherein the inner shaft is configured for rotation and the outer shaft is configured for deflection.

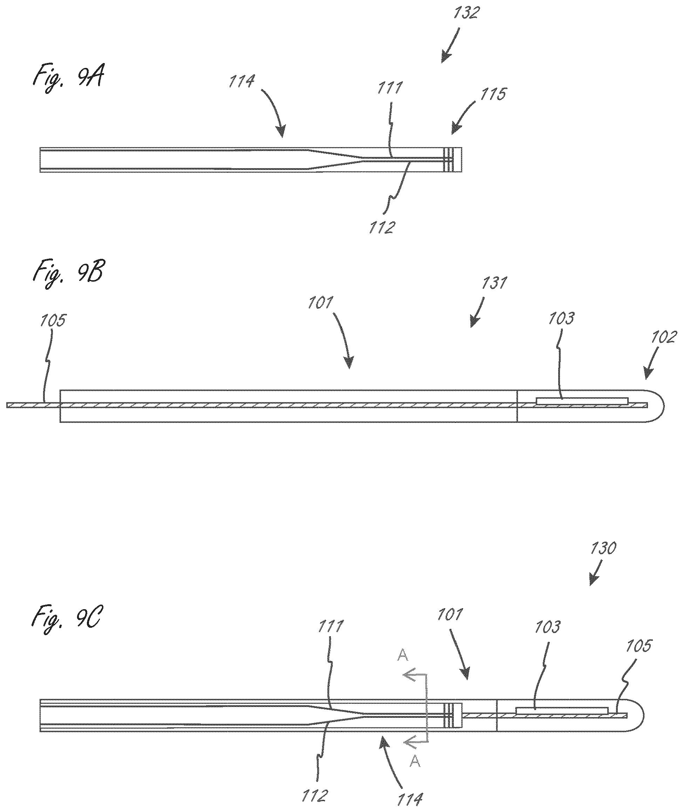

Figure 9C shows example apparatus medical apparatus 130, which includes elongate inner shaft 132 (see figure 9A) and elongate outer shaft 131 (see figure 9B). Medical apparatus 130 may also be referred to herein as a "catheter," or other medical device that includes at least one elongate shaft.

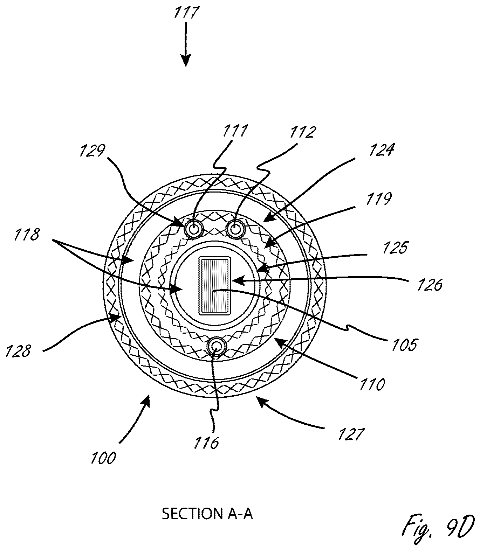

Figure 9D illustrates Section A-A shown in the assembly in figure 9C, which is a section in the deflectable section of the device. Parts from figures 9A-9C are similarly labeled. As can be seen in figure 9D, pull wires 111 and 112 are very near to one another and about 180 degrees away from straightening pull wire 116.

As is also shown in figure 9D, elongate inner shaft 132 includes two layers of braided material 119, and the pull wires are, at least at the location of this section, essentially sandwiched between the two layers of braided material. Annular spaces 118 allow freedom of movement and space for optional lubricant. Inner shaft 132 may be made from, for example without limitation, a polymeric material such as Pebax, optionally with a lubricious additive. Inner shaft 132 may include liner 125, such as a PTFE liner. The flexible cable bundle 105 may be surrounded by one or more layers of insulation 126, such as PTFE insulation. Outer shaft 131 may comprise a polymeric material 127, such as Pebax. Outer shaft 131 may also include a radially inner liner 128, such as a PTFE liner. Any of the pullwires (e.g., 111, 112, 116) may be disposed in a lumen with a liner, such as PTFE liner 129.

Medical apparatus 130 (or either of elongate shaft 132 and elongate shaft 131, individually) can be in operable communication with any of the handle assemblies herein, including handle assembly 120 shown in figures 8A and 8B.

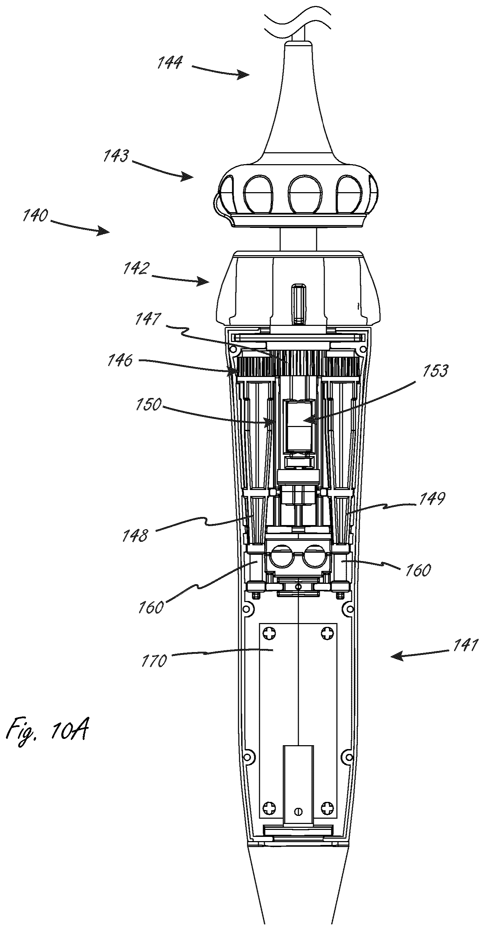

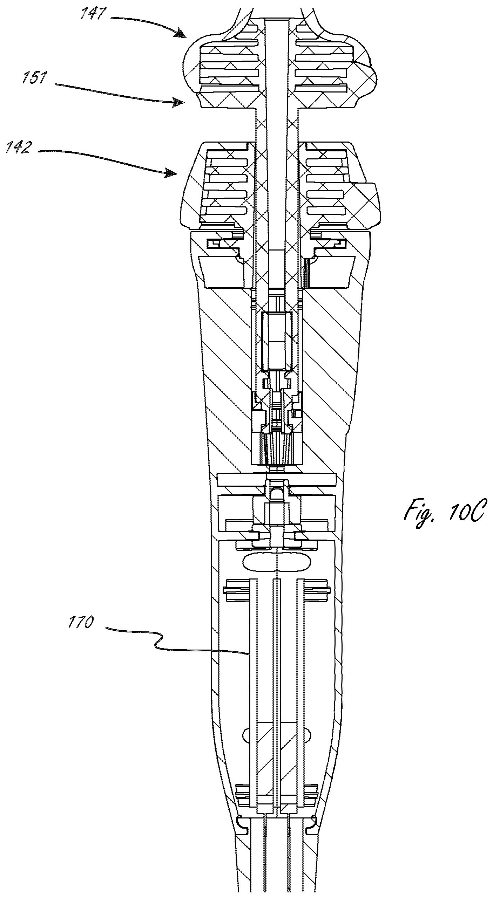

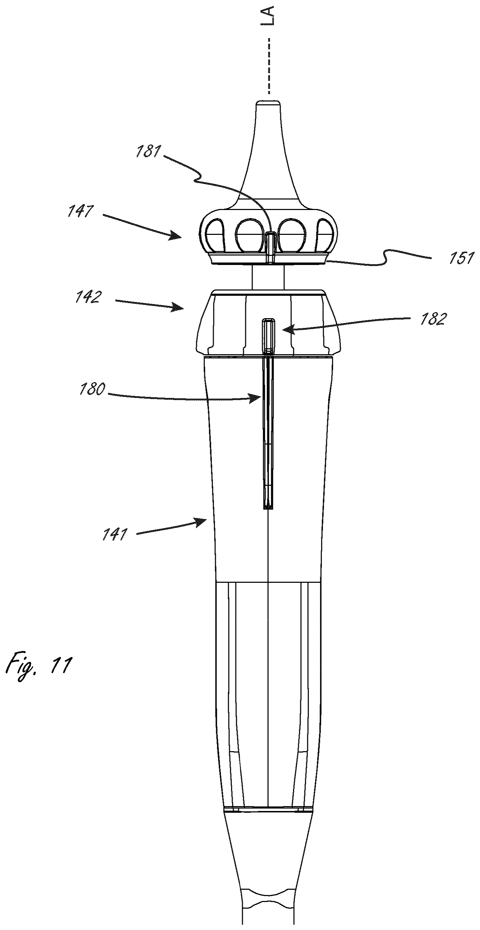

Figures 10A-10C and figure 11 illustrate an additional example handle assembly that can be in operable communication with any of the medical devices, including ultrasound probes, herein. For example, the example handle assembly shown in figures 10A-10C can be coupled to (directly or indirectly) and in operable communication with medical apparatus 130 shown in figures 9A-9D. In a particular embodiment, both elongate outer shaft 131 and elongate inner shaft 132 are coupled to and in operable communication with the handle assembly shown in figures 10A-10C.

The handle assembly in figures 10A-10C and figure 11 has some similarities to the handle assemblies, the individual components, and subassemblies that are shown in figures 8A and 8BB. Unless indicated to the contrary, concepts, features and methods of use from figures 8A and 8B that can be incorporated into the handle assembly in figures 10A-C are hereby incorporated by reference for all purposes into the disclosure of the handle assembly shown in, and described with respect to, figures 10A-C. Similarly, concepts, features and methods of use that are shown in, and described with respect to, figures 10A-C that can be incorporated into other handle assemblies herein are hereby incorporated by reference for all purposes into the disclosure of any of the handle assemblies set forth herein.

Figure 10A is side view of handle assembly 140 with a portion of handle body 141 removed so that some internal components of the handle assembly can be seen. Handle assembly 140 includes first actuator 143 and second actuator 142, and in this embodiment first actuator 143 is distal to second actuator 142. First actuator 143 can be both moved axially and rotated relative to the handle body and relative to a second actuator (in this embodiment actuator 142). First actuator 143 is in operable communication with an outer elongate body, such as outer shaft 131 (*see* figure 9B). Axial movement of actuator 143 (distally or proximally) causes axial movement of the outer shaft 131, while rotation of actuator 143 causes rotation of the outer shaft. Second actuator 142 is in operable communication with an inner shaft, such as inner shaft 132 (figure 9A). Actuation of second actuator 142, in this embodiment rotation, causes deflection of the inner shaft. In this embodiment a rotatable and axially movable actuator (i.e., first actuator 143) is in operable communication with an outer shaft. Although reference is made that actuation of second actuator 142 may cause deflection of the inner shaft, alternatively, actuation of second actuator 142 may cause rotation of the inner shaft. Likewise, actuation of actuator 143 may alternatively cause deflection of the outer shaft.

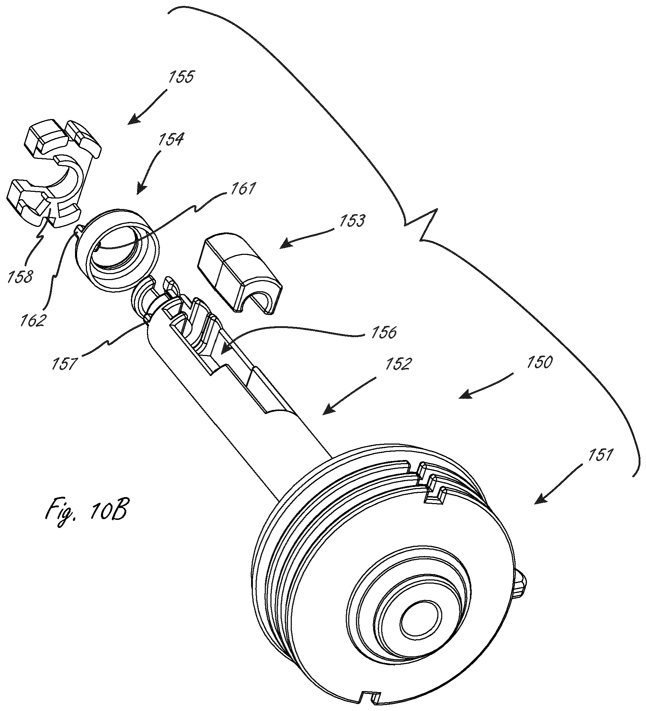

First actuator 143 is coupled to elongate outer shaft movement assembly 150 shown in the exploded view in figure 10B, such that movement of first actuator 143 causes movement of assembly 150. Elongate outer shaft movement assembly 150 is similarly coupled to the elongate outer shaft so that movement of first actuator also causes movement of the elongate outer shaft. In this embodiment, the outer elongate shaft is attached to removable part 153 after it is inserted into channel 156. Removable part 153 and channel 156 are configured so that removable part 153 is constrained by at least one inner surface of channel 156 when it is inserted therein. Elongate outer shaft movement assembly 150 also includes a distal head portion 151 that is secured to first actuator. Elongate outer shaft movement assembly 150 also includes a rotation limiting mechanism similar to that which is described herein, which limits the rotation of first actuator 143, and thereby limits the rotation of the outer elongate shaft. Any of the disclosure above related to rotation limiting subassemblies, functionality, and use, is incorporated into this embodiment for all purposes and may be incorporated into this and similar designs. During rotation, part 157 (see fig. 10B) interacts with part 161, and part 162 interacts with part 158. The physical interactions of these two sets of parts limits rotation to the desired rotation limit, e.g., such as limiting rotation up to 630 degrees of rotation of the outer body (in other embodiments the allowed rotation could be more than 630 degrees, such as up to and including 720 degrees).

If it is desired to clean the outer shaft, for example after use, removable part 153 can be detached from the outer shaft to allow the outer shaft to be removed from the handle assembly and cleaned, before being reinserted and reattached to removable part 153 or a new removable part if part 153 is damaged or broken.

Handle assembly 140 also includes inner shaft deflection assembly 146, which is in operable communication with second actuator 142. Inner shaft deflection assembly 146 includes central gear 147 adapted and configured to rotate when second actuator 142 is rotated. Central gear 147 interfaces first spindle 148 and second spindle 149 via a geared interface, such that rotation of central gear 147 causes rotation of the spindles in the opposite direction. The inner shaft deflection assembly 146, including the spindles, extends further proximally than the elongate outer body movement assembly 150. The inner shaft extends through the outer shaft and extends further proximally than the outer shaft within handle assembly 150. This allows one or more pull wires that are part of the inner shaft to extend radially outward and interface with reels 160.

Although the central gear 147 and the geared interfaces are shown with spur gears, in other embodiments helical gears may be used. Helical gears may provide for smoother control and reduced slop of the first actuator 143 and the second actuator 142. The helical gear design may provide smoother operating performance than spur gears. The helical gears may have a larger number of "virtual teeth" when compared to physical teeth. The helical gears may have the same number of "virtual teeth" as spur gears have physical teeth and "virtual teeth", while having less physical teeth than the spur gears, allowing the device to have the same or better functionality while using a smaller area. The helical gears may have stronger teeth, as measured by bending and surface fatigue, when compared to spur gears with the same number of physical teeth. The helical gears may reduce undercutting, allowing for fewer physical teeth when compared to a minimum number of physical teeth needed for spur gears. An example helical gear configuration may comprise twenty-six (26) gear teeth, seventeen (17) pinion teeth, a twenty-six to seventeen (26:17) gear ratio, a twenty degree (20°) pressure angle, and a twenty degree (20°) helix angle. An example helical gear configuration may comprise one point zero degree (1.0°) gears meshed to align, allowing for easy manufacturability. The helical gears may be molded. The helical gears may comprise a configuration similar to spur gears. The helical gear configuration may comprise spindles, such as the first spindle 148 and the second spindle 149, comprising thru holes, as spindles would have in a spur gear configuration.

The lack of interaction between elongate outer shaft movement assembly 150 and elongate inner shaft movement assembly 146 allows for the inner and outer elongate shaft to be independently controlled by first actuator 143 and second actuator 142.

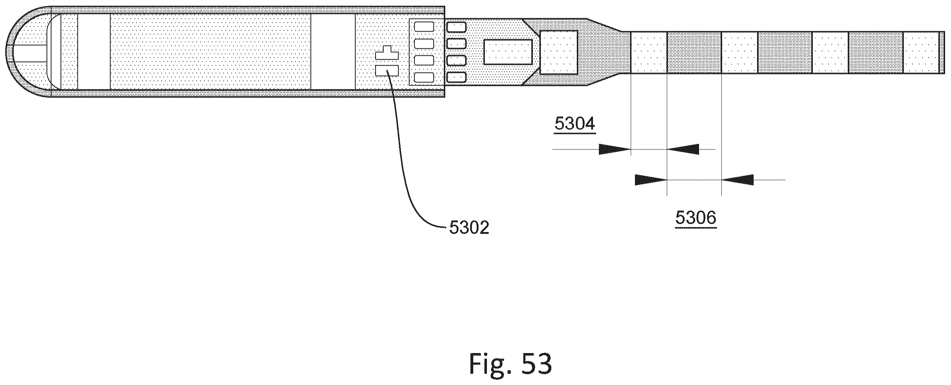

Handle assembly 140 also includes printed circuit board ("PCB") 170 disposed within handle body 141, the PCB being in electrical communication with a cable bundle, such as flexible cable bundle 105 in figure 53, or any of the cable bundles herein that are in communication with the medical tool, such as an ultrasound transducer.

Handle assembly 140 also includes a rotation indicator 180 that can be used to show a user the extent to which at least one of the first actuator and the second actuator are rotated relative to a home, or neutral position. First actuator 143 can include a rotation indicator 181 that is aligned along an axis with rotation indicator 180 when first actuator 143 is in a neutral position, as shown in figure 11. When first actuator 143 is rotated, rotation indicator 181 is rotated relative to the axis along which rotation indicator 180 extends, which enables the user to visually understand that first actuator 143, and thus the outer shaft, is rotated to some extend relative to the neutral position. Similarly, second actuator 142 can also have rotation indicator 182 that is aligned along an axis with rotation indicator 180 when second actuator 142 is in a neutral position, as shown in figure 11. When second actuator 143 is rotated, rotation indicator 182 is rotated relative to the axis along which rotation indicator 180 extends, which enables the user to visually understand that second actuator 143, and thus the inner shaft, is deflected to some extent relative to its neutral position.

In some alternative embodiments, the handle assembly can include one or more sensors to track how much rotation has occurred for the outer shaft, or how much deflection has occurred in the inner shaft. In some embodiments the handle assembly can include an encoder for each actuator.

In any of the embodiments herein that include an outer shaft and an inner shaft, the device can include one or more lubricants between the inner and outer shafts to make it easier to move the inner and outer shafts relative to one another by reducing friction between the two. If the medical device needs to be cleaned for reuse, additional lubricant can be added between the inner and outer shafts after the cleaning process.

In some embodiments herein the medical device may include a flexible member, such as a flexible conductor bundle (which may be referred to herein as a conductor bundle, flex bundle or other similar derivative thereof), coupled to and extending from a distal region (e.g., probe tip) towards a proximal region of the medical device (see, for example, conductor bundle 2020 shown in example figures 4-6G; or bundle 105 from figure 9B). A probe tip may include an ultrasound transducer in electrical communication with a flexible conductor bundle. In some embodiments herein (e.g., figures 9A-11), the probe tip and conductor bundle can be axially displaced (proximally and/or distally) by actuation of a handle actuator (e.g., actuator 143 as shown in figure 10A). In some instances, the conductor bundle is disposed within an elongate member (e.g., steerable inner elongate body 132; or elongate member 131) and moves axially relative thereto when the probe tip is advanced distally or retracted proximally. When the distal region (e.g., ultrasound probe) and conductor bundle are retracted proximally (after being advanced distally), the conductor bundle may tend to fold up, bunch up, or otherwise bend, near or adjacent to its distal end due to friction between the bundle and, for example, the elongate member (e.g., steerable inner shaft 132) in which the conductor bundle is disposed. Bunching may occur if the medical device is in a straight configuration as well as if the medical device has some degree of bend (e.g., after being deflected from a straight or linear configuration).

To reduce the degree of, or even prevent completely, a tendency to bunch up or bend, any of the medical devices herein may include a structural tensioning member that is adapted and configured to apply or maintain tension on the flexible member, such as a flexible conductor bundle, at a location that is proximal to where the conductor bundle is coupled to the distal medical tool. By tensioning the flexible member, folding or bunching up of the flexible member can be minimized or even prevented. When used in this context, the "tensioning" member is adapted and configured to reduce distal region(s) of the flexible conductor from bunching (compared to a device without a tensioning member) by proximally moving at least a distal portion of the flexible conductor bundle as the distal probe is retracted proximally. In some embodiments, the structural tensioning member (e.g., a tensioning bar) may be physically secured to the flexible conductor bundle (e.g., direct or indirect attachment). In general, the tensioning members herein are in operable communication with the flexible members (e.g., a flex conductor bundle) such that movement or actuation of the tensioning member applies some force to the flexible member, and may cause movement (e.g., proximal) of the flexible member. In some example embodiments the structural tensioning member may be disposed in or carried by the handle assembly of the medical device. Structural tensioning member in this context may be a single component or an assembly of separate components.

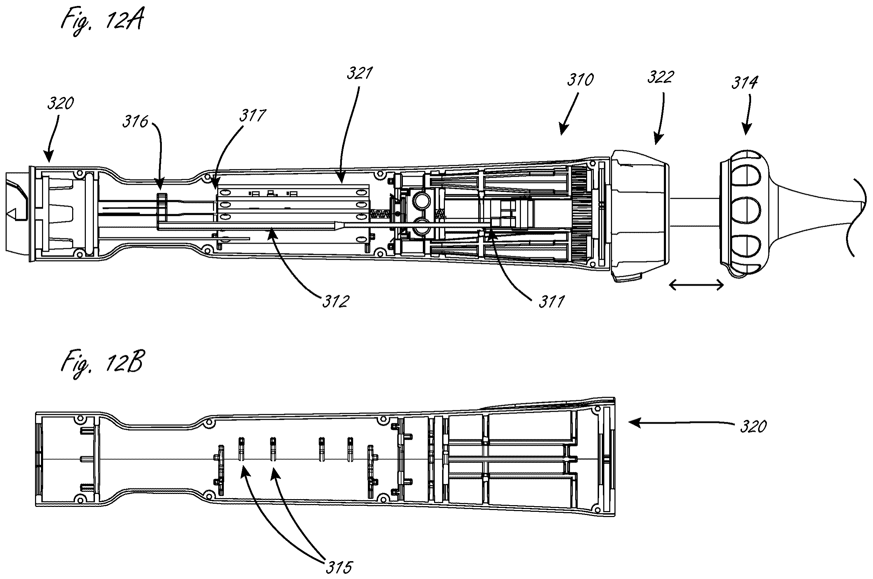

Figures 12A-12F illustrate a handle assembly portion of an example medical device, which may be incorporated into any suitable medical device herein. For example, the handle assembly in Figures 12A-12F may be part of a medical device that includes a medical tool (e.g., an ultrasound imaging probe) at its distal end or near its distal end, examples of which are described herein. Any other embodiment or feature herein is incorporated by reference into the example handle assembly shown in figures 12A-12F.

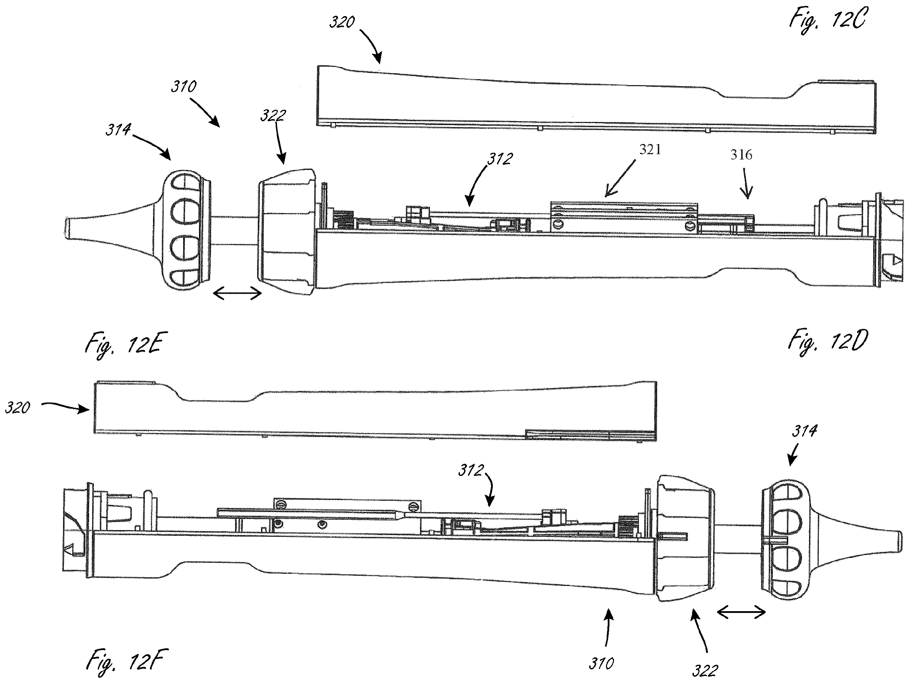

Example handle assembly 310 includes first actuator 314 and second actuator 322, where first actuator 314 is distal to proximal actuator 322. First actuator 314 is adapted and configured to be moved axially (distally and proximally) relative to second actuator 322 (and optionally also rotatable relative thereto), and may be in operable communication with an elongate body (e.g., 131 or 132) that may include a medical tool (e.g., 103) in a distal region. Handle assembly 310 (including actuators) may incorporate any of the relevant disclosure from any other handle assembly herein. The medical device of which handle assembly 310 is a part also includes a flexible member (e.g., flexible conductor bundle 105 in figure 9B) securely coupled to (directly or indirectly) the medical tool at a first distal location (e.g., as shown in figures 9B and 9C where medical tool 103 is secured to flexible member 105) and extends proximally from the medical tool towards handle assembly 310. The flexible member may be a flexible conductor bundle and can extend into handle assembly 310, as shown. A portion of the flexible member is disposed within an outer surface of the elongate body (e.g., within 131 and/or 132). The medical device also includes tensioning member that is secured to the flexible member at a second location 316, which is proximal to the first location ("first location" in this context may be referred to as a first distal location or derivative thereof). Figure 12A illustrates example tensioning member 312, while reference number 312 in figure 12A also points to an optional elongate rigid member (in this embodiment the elongate rigid member is linear and extending axially) of the tensioning member. Tensioning member 312 is adapted and configured to tension the flexible member as the medical tool is retracted proximally. This may be referred herein as applying a tensile force to the flexible member, or tensioning the flexible member, or maintaining tension in the flexible member. In this example embodiment, tensioning member 312 is adapted and configured to tension the flexible member as the medical tool is retracted proximally, which in this embodiment occurs when first actuator 314 is retracted proximally from its position shown in figures 12A, 12D, and 12F. The tensioning members herein can apply tension when the medical device is in a straight configuration and when the medical device is in a non-straight (linear) configuration, such as when the device may be deflected or bent.

In this embodiment, tensioning member 312 (which may include the tensioning bar shown in figure 12A) is secured to (e.g., attached directly) the flexible conductor bundle at location 316, the location of which is inside the handle assembly in this embodiment. A tensioning member may alternatively be secured to the flexible member at a location that is not within a handle, such as outside of the handle, such as inside one or both of outer and inner shafts. In this embodiment the tensioning member is also axially secured relative to first actuator 314, such that axial movement of first actuator 314 causes axial movement of tensioning member 312 (which may be in a 1:1 movement ratio). Because tensioning member 312 is also secured to the flexible member (e.g., at location 316), axially movement of tensioning member 312 also causes axial movement of the flexible member at location 316. By securing tensioning member 312 to the flexible member, the flexible member is tensioned distal to where the tensioning member is secured to the flexible member when first actuator 314 is retracted proximally, which prevents the flexible member from folding or bunching up at its distal region near or adjacent to the medical tool (or at least reduces the extent of folding/bunching up compared to a device without a tensioning member).

The flexible member may include a flexible conductor bundle, such as any of the flexible conductor bundles herein. In figures 12A-F, the tensioning member comprises a rigid elongate member (generally referred to as 312 in figure 12A) with a fixed length. The rigid tensioning member as shown has a general longitudinal axis, which in this embodiment is parallel with a longitudinal axis of the medical device and/or a longitudinal axis of the handle assembly. The rigid tensioning member may be made of a variety of materials, such as a rigid plastic member.

The tensioning members herein can ensure that the distance traveled by the medical tool is the same as the distance traveled by any point on the flexible member between the first and second locations. The tensioning members herein can ensure that the distance traveled by the medical tool is the same as the distance traveled by the location where the tensioning member is secured to the flexible member (e.g., location 316 in figure 12A).

The secured relationship between the tensioning member and the flexible member maintains the flexible member in a substantially flat or straight configuration between the first and second locations as the medical tool is retracted proximally (when the medical device is a straight configuration). A flat configuration in this context can include embodiments in which the flexible member may also be twisted (i.e., the flexible member can be flat and still be twisted, but not bunched/folded). A flattened configuration as used herein indicates a lack of a fold or bunching of the flexible member.

Medical devices in which a tensioning member is incorporated may also be steerable or deflectable. The tensioning members herein can be adapted and configured to apply a tensile force to the flexible member even if the medical device, including the flexible member, are in non-straight (e.g., deflected, steered, bent) configurations. When this disclosure refers to maintaining a substantially flat configuration in the flexible member, it refers to instances where the medical device may be in a straight configuration, which need not be the case, such as when a medical device has been steered, bent, or deflected.

The secured relationship between the tensioning members and the flexible members herein prevents the flexible member from forming a fold (i.e., bending, or bunching up) between the first and second locations as the medical tool is retracted proximally. Folding, bending, and bunching-up in this context includes a first region of the flexible member axially overlapping with a second region of the flexible member, and also includes general bending and bunching of the flexible member, such as regions of the flexible member that are not flat and, for example, form a bend, curved region, and/or meander back and forth.

The flexible member may have flat top and bottom surfaces (e.g., a conductor bundle with one or more flat surfaces), and optionally the tensioning member may be secured to at least one of the top and bottom surfaces. For example, figures 6A-6G illustrate a flexible member with flat or generally flat first and second surface (e.g., top and bottom surfaces), and a tensioning member may be secured to one or both of the flat or generally flat surfaces (e.g., such as at location 316). They may be secured using a variety of techniques, such as using adhesive, welding, or other bonding techniques.

The tensioning member may be in operative communication (directly or indirectly) with a handle actuator such that axial movement of the actuator axially moves the tensioning member. The handle actuator may be further adapted and configured to be rotated (e.g., actuator 314) to cause rotation of the medical tool, and optionally wherein rotation of the actuator does not cause rotation of the tensioning member. The tensioning member can thus be adapted to be moved axially when the actuator is moved axially, but not to rotate when the actuator is rotated. This can be accomplished by the manner in which the tensioning member is operatively in communication (directly or indirectly) with the actuator.

The medical device may include an inner elongate body (e.g., 132) including a lumen in which at least a portion of the flexible member is disposed. An inner elongate body may be independently steerable, such as with a separate independently actuatable handle actuator (e.g., actuator 322). Aspects of other embodiments herein in which the medical device includes an inner member and outer member, the inner member independently controllable (e.g., axially and rotationally) are fully incorporated in any of the embodiments herein.

The flexible member may be coupled to a printed circuit board (e.g., 321 "boards" as shown in figure 12A) in the handle assembly. The tensioning member may be coupled to a flexible member proximal to a printed circuit board. In alternative embodiments the tensioning member may be coupled to a flexible member distal to a printed circuit board.

Figures 12A-12F is an example of a portion of a medical device that includes an elongate outer body (e.g., 131) including a probe tip in a distal region of the elongate body; a flexible conductor bundle (e.g., 105) securely coupled to the probe tip at a first location (shown in figure figures 9B and 9C) and extending proximally from the medical tool and into a handle assembly, the flexible member disposed within an outer surface of the elongate body; an inner elongate body (e.g., 131), at least a portion of which is disposed within the elongate outer body, the inner elongate body optionally steerable, wherein at least a portion of the flexible conductor bundle is disposed within the inner elongate body and configured to be axially movable relative to the inner elongate body; a tensioning member secured to the flexible member at a second location in the handle assembly, the tensioning member adapted and configured to apply tension on the flexible member as the probe tip is retracted proximally. Probe tips as used herein may include one or more ultrasound transducers.

Figures 12C and 12E illustrate a half of a handle outer shell 320, two of which form a portion of the outer surfaces of the handle assembly 310. Handle shells 320 include radially inwardly extending features 315 that are adapted to interface with a control the movement of the tensioning member 312. Features 315 can include guides that are configured to interface with the tensioning member at one or more locations and help stabilize the tensioning member.

Any other handle assembly component in any other embodiment herein that can be suitably integrated into handle assembly 310 is incorporated by reference herein.

In any of the embodiments and claims herein, the phrase "tensioning member" may be replaced with "straightening member," "flattening member," or a derivative thereof. As described herein, a straightening member or flattening member refers to maintaining a substantially straight or flattened configuration in the flexible member when the medical device is in a straightened configuration, and does not require that the device always has a straightened configuration. Thus, even if the flexible member is not necessarily being placed under tension, it may still be maintained in a straightened (i.e., not folded configuration) along at least a portion of its length due to a straightening member. Member 312 in figure 12A, for example, is an example of a straightening member, even if it also functions as a tensioning member. This applies to all tensioning members described, shown, and claimed herein. In some embodiments herein, the "member" can be a straightening member (or flattening member) and can also function as a tensioning member. Additionally, the phrase "tensioning member" herein may be replaced with "fold-prevention member," "bend-prevention member," "bunching-prevention member," or a derivative thereof.

The disclosure above describes that in some embodiments the flexible member, such as conductor bundle 2010, may be twisted along a portion of its length. For example, a conductor bundle may be twisted to provide a more balanced cross-section along a portion of the length of the medical device. A conductor bundle may be twisted only in a portion of the medical device that will experience deflection.

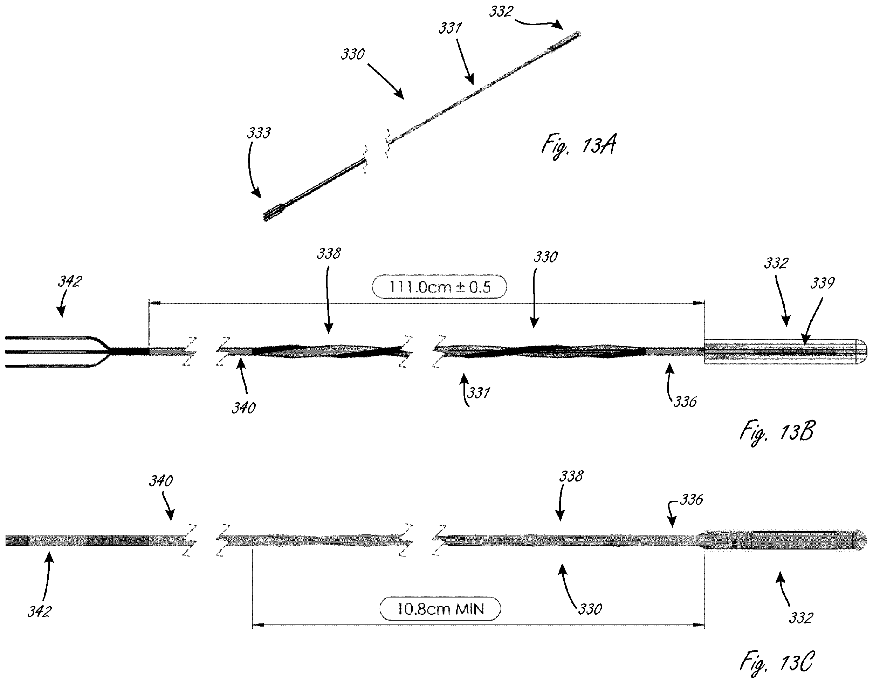

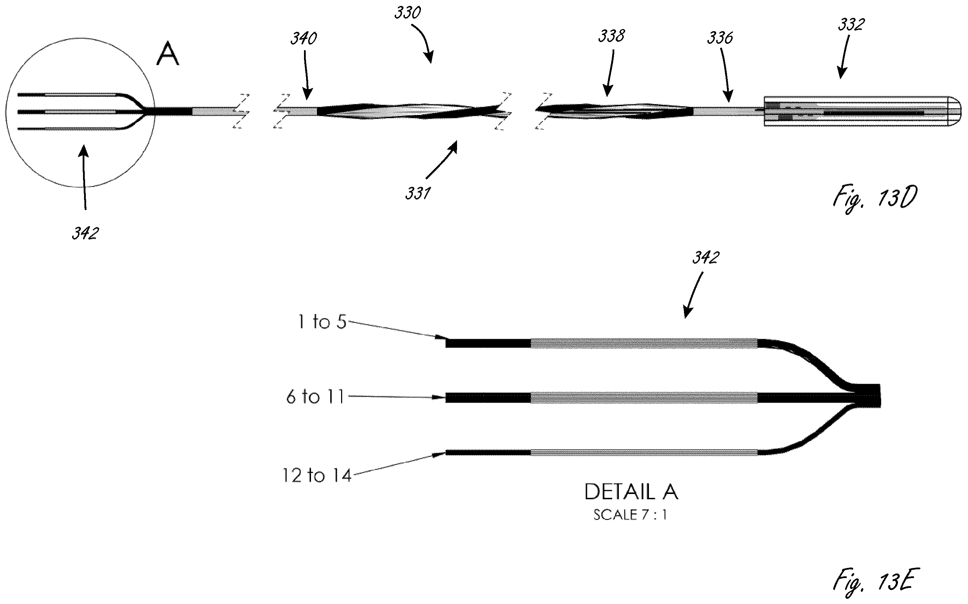

Figures 13A-13E illustrate a portion of an example medical device that includes a twisted flexible member, such as a flexible conductor bundle. The embodiment in figures 13A-E may be incorporated with any other suitable feature and/or medical device described herein.

The portion 330 of the medical device shown in figures 13A-E includes a medical tool 332 at a distal region, a flexible member 331 coupled thereto and extending proximally therefrom, and a proximal end region 333 comprising a plurality of electrical connectors. Flexible member 331 may be a flexible conductor bundle, such as any of the bundles herein. Medical tool 332 may include an ultrasound imaging transducer 339. Flexible conductor bundle 331 has a region 338 in which the conductor bundle is twisted, the twisted region 338 having a distal end and a proximal end. Flexible conductor bundle 331 also includes a region 336 distal to twisted region 334 that is not twisted, and a region 340 proximal to twisted region 336 that is not twisted. Medical tool 332 may be coupled to an outer shaft, such as outer shaft 131 shown in figure 9B.

The length of twisted region 338 from its distal end to its proximal end can vary, and in some embodiments is from 5-15 cm, such as from 8-15 cm, such as 11 cm. The length over which a complete turn is formed can vary well, such as from 1-5 cm, such as 3 cm.

The number of twists over the length of the twisted region can vary as well, such as, for example without limitation, 7-9 full twists.

An example manner in which to form the twisted region of the flexible member (e.g., flexible conductor bundle) is to couple the flexible member to the medical tool at the distal end (e.g., probe tip). A thin section of PET heat shrink may then be advanced over the flexible conductor bundle. A portion of the device can be held in place while another portion is twisted to the desired number of turns to form the twisted region. While the twisted configuration is maintained, the PET can be heat shrunk over the twisted bundle region. Additional layers of PET may be subsequently added. An elongate member (e.g., shaft 131) may then be placed over the bundle, including the twisted region, and the elongate member can be bonded to the medical tool.

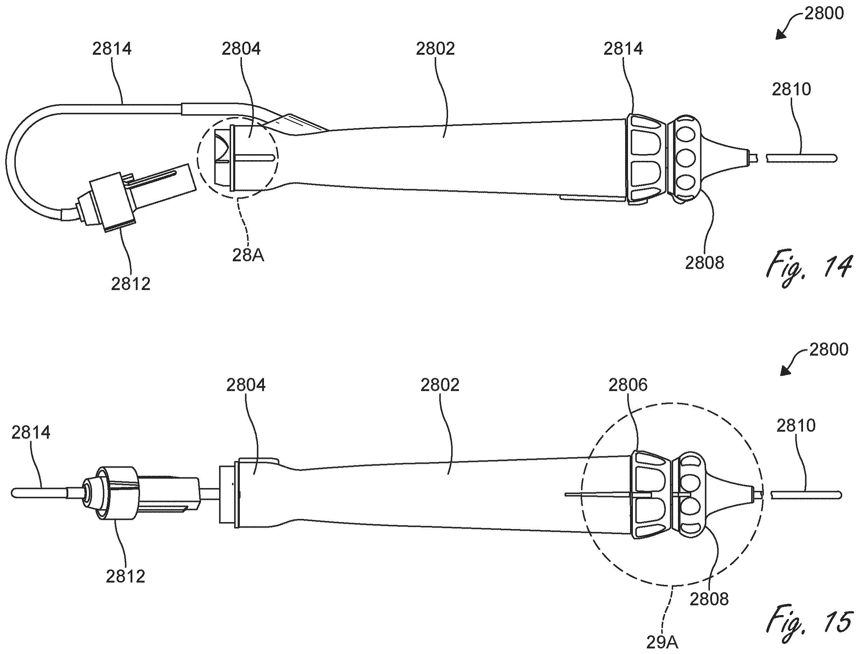

A Referring to FIGS. 14-18, a system 2800 includes a handle assembly 2802 and steering and medical device portion 2810 (e.g., shown as an outer sheath similar to sheath 1208 (FIG. 4), which may further enclose an inner shaft and tool. The system is adapted so that handle assembly 2802 can be actuated to cause steering of a steerable portion of the steering and medical device portion 2810, and optionally can be further actuated to cause movement of a medical device coupled thereto. As an illustrative example, the handle assembly 2802 includes a first actuator 2806 and a second actuator 2808. One or more of the first actuator 2806 or the second actuator 2808 is adapted (e.g., configured) to be actuated (in this example rotated) relative to a handle body of the handle assembly 2802 to cause the steering of steerable portion 2810, such as steering an outer sheath. Steering may include rotating or deflecting. As an example, one of the actuators 2806, 2808 controls rotation, while another of the actuators 2806, 2808 controls deflection. As a further example, one of the actuators 2806, 2808 controls rotation of one or more of the inner shaft or outer shaft, while another of the actuators 2806, 2808 controls deflection of one or more of the inner shaft or the outer shaft.

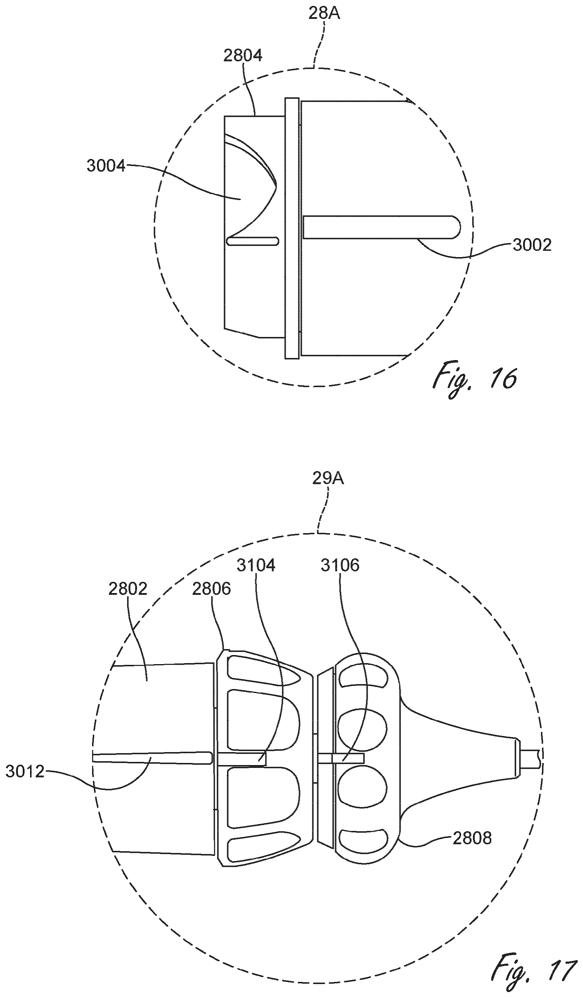

As more clearly shown in FIG. 17, a neutral position marker 3102 may be disposed on a body of the handle assembly 2802 to indicate (e.g., visually, tactilely, etc.) a fixed reference point. One or more of the actuators 2806, 2808 may further include a marker 3104, 3106 to indicate alignment or position of the actuators 2806, 2808 relative to the neutral position marker 3102. As an example, the marker 3104 may indicate a deflection of the shaft relative to the neutral position marker 3102 and the marker 3106 may indicate a rotation of a component (e.g., transducer face disposed in the sheath 2810) relative to the neutral position marker 3102, or vice versa. The markers 3104, 3106 may indicate other positions. Additionally or alternatively, mechanical registration mechanisms, such as detents, may be used to provide tactile feedback to a user to indicate certain positions of the actuators 2806, 2808, such as when the actuators 2806, 2808 are in the neutral position.

The handle assembly 2802 may include any other handle component or functionality described in any of the other handles herein. For example, a receptacle 2804 may be disposed at a proximal end of the handle assembly 2802 and adapted to receive a plug such as an umbilical plug. As more clearly shown in FIG. 16, the receptacle 2804 may comprise visual and/or tactile orientation markers for registration and alignment of an umbilical plug and the receptacle 2804.

A navigation connector 2812 may be coupled to the handle assembly 2802 via a navigation conduit 2814 (e.g., navigation cable sleeve). The navigation connector 2812 may be configured to interface with a navigation system such that the system 2800 may communicate with the navigation system. As an example, the navigation system may provide tracking and navigation of a portion of the system (e.g., the medical device) while in use. As shown, for example, the navigation conduit 2814 may couple to a body of the handle assembly 2802 to provide access for one or more cables to pass from the navigation connector 2812 to components within the housing of the handle assembly 2802. As more clearly shown in FIG 18, the navigation connector 2812 may be configured to interface with a navigation system connector 3204 (e.g., receptacle), which may be proprietary to the navigation system manufacturer. Other configurations may be used.

FIGS. 19-24 illustrate an example distal region of a steerable system that includes a medical tool. As an illustrative example, FIG. 19 shows the medical tool may comprise a steerable tip 3300 disposed at the distal end of a sheath. The steerable tip 3300 may include a transducer 3304 (e.g., ultrasound transducer) at least partially enclosed by a material 3302 (e.g., polymer). The tip 3300 may comprise an electronic module 3306, which may include sensors, control boards, thermistors, and the like. As an example, a triple axis sensor (TAS) 3308 may be disposed in communication with the module 3306. As a further example, a sensor cable bundle 3310 may be disposed to provide electrical communication to one or more components coupled to the module 3306.

As an illustrative example, FIG. 20 shows the medical tool may comprise a steerable tip 3400 disposed at the distal end of a sheath. The steerable tip 3400 may include a transducer 3404 (e.g., ultrasound transducer, folded transducer) at least partially enclosed by a material 3402 (e.g., polymer). The tip 3400 may include sensors, control boards, thermistors, and the like. As an example, a triple axis sensor (TAS) 3408 may be disposed in in a configuration that is closer to the distal end of the tip 3400, when compared to the tip 3300 configuration in FIG. 19. By shifting the sensor 3406 the proportion of the tip 3400 that may be deflected is increased.

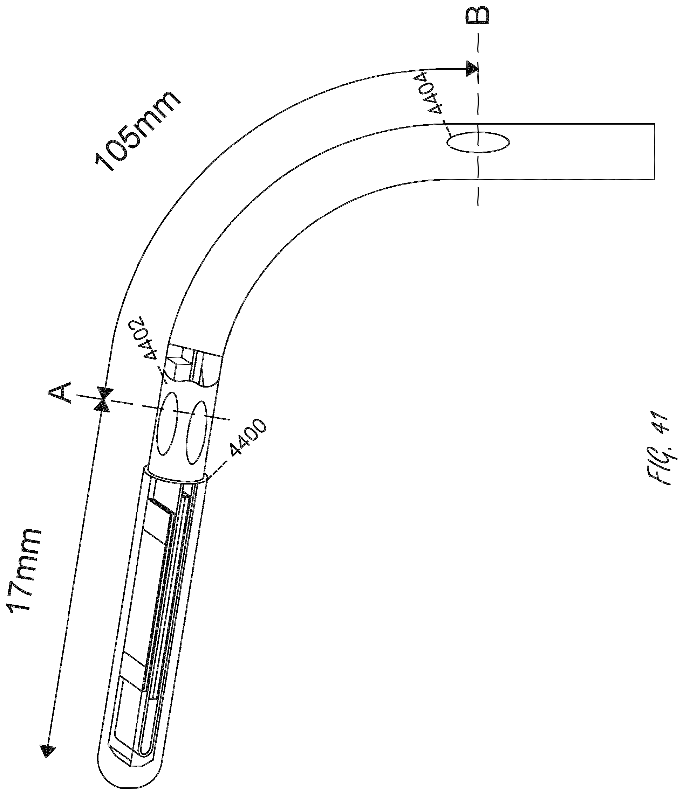

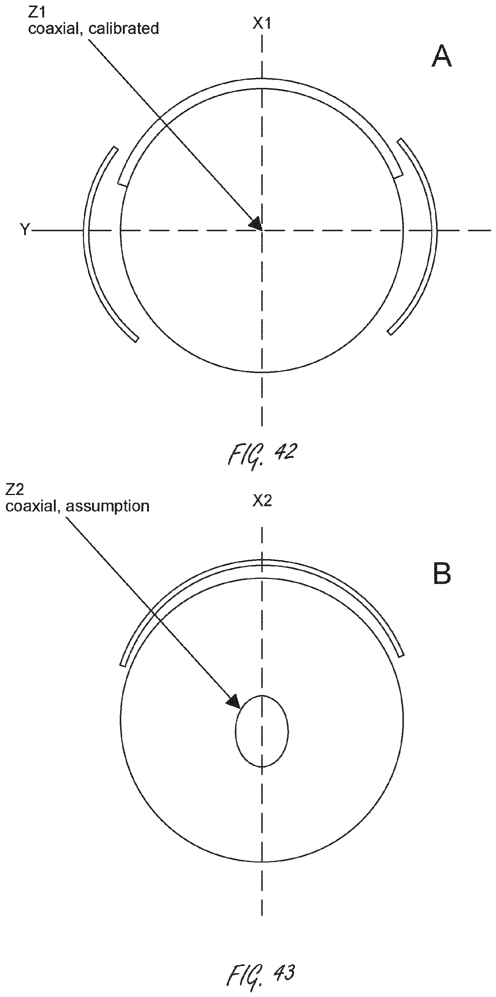

Although FIG. 19 and FIG. 20 shows the TAS sensor 3308, in other embodiments, single axis sensors (SAS) or double axis sensors (DAS) may be used. Replacing the TAS sensor 3308 with a SAS or a DAS may reduce a length associated with the tip 3300 and a post length, allowing for more distal deflection. As an illustrative example, FIGS. 41-43 illustrate how the use of a DAS 4402 at or adjacent the tip portion 4400 (e.g. 17mm from the distal extent of the tip) and a SAS 4404 at some distance from a distal end of the inner shaft terminating into the tip portion 4400 (e.g., 105mm from the DAS 4402), a projection on tip rotation and shaft curvature may be calculated. As shown, Fig. 42 is a cross section taken across line A and Fig. 43 is a cross section taken across line B in Fig. 41. For example, an angle between X1 and X2 about Z may be used to project independent tip rotation. As a further example, an angle between Z1 and Z2 may be used to project shaft curve. The DAS 4402 is preferably coupled to a proximal extension of the transducer tip (e.g., at an end of the tip portion 4400), which is also coupled with the outer shaft, such that the DAS 4402 moves together with the outer shaft and tip. The SAS 4404 is preferably coupled to the inner shaft. The DAS may therefore move independently of the SAS 4404. The SAS 4404 location on the inner shaft is preferably proximal to the deflection location. The location may also be preferably located on a stiffer portion of the inner shaft, proximal to both the deflection region and a softer portion of the shaft just proximal to the deflection region. The SAS 4404 location may be in a range of 5-15 cm from the distal end of the inner shaft.

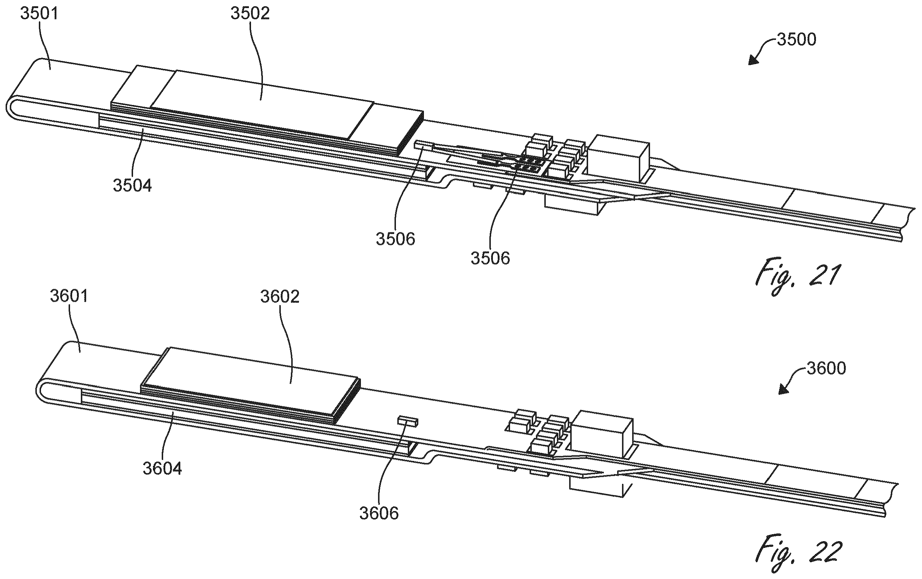

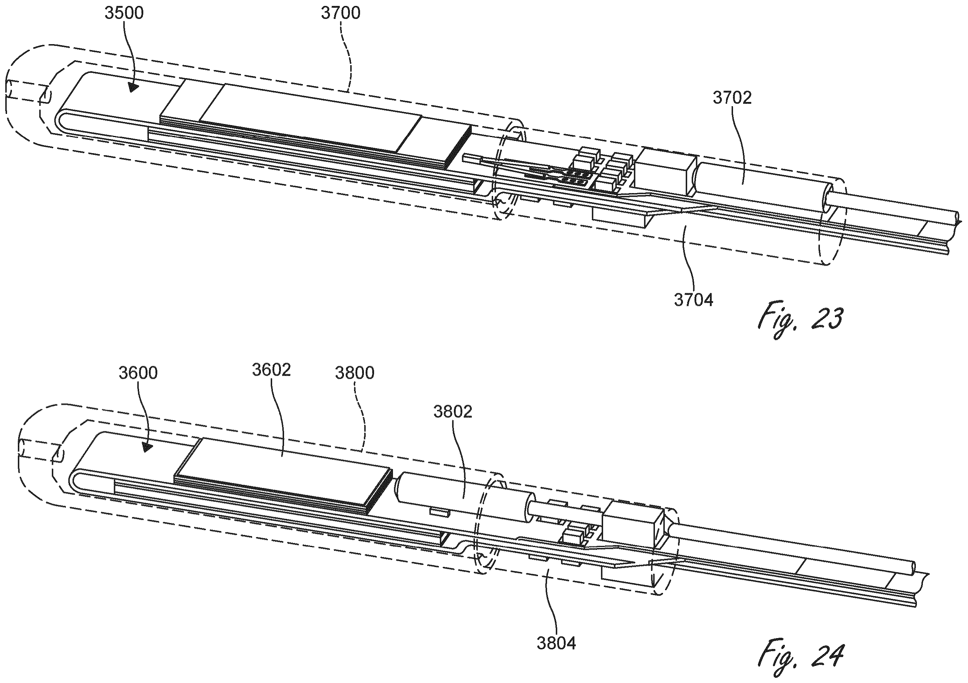

As an illustrative example, FIG. 21 shows the medical tool may comprise a steerable tip 3500 disposed at the distal end of a sheath. The steerable tip 3500 may include a transducer 3502 (e.g., ultrasound transducer, folded transducer). As an example, an ASIC 3504 may be disposed adjacent the transducer 3502. As a further example, inactive piezo elements may be disposed adjacent the transducer 3502. The tip 3500 may include various sensors, control boards, thermistors, and the like. As shown, the tip 3500 includes thermistors 3506 disposed proximal to the transducer 3502. As more clearly shown in FIG. 23, at least a portion of the tip 3500 may be at least partially enclosed by a first material 3700 (e.g., polymer) and at least a portion of the tip 3500 may be at least partially enclosed by a second material 3704. The first and second materials 3700, 3704 may be configured with the same or different characteristics such as differing stiffness (e.g., durometer). As an illustrative example, . As further illustrated in FIG. 23, a navigation sensor 3702 may be disposed at or adjacent a proximal end of the tip 3500. The navigation sensor 3702 may be in communication with a navigation system to provide tracking and position of the tip 3500 and to provide feedback to a user.

As an illustrative example, FIG. 22 shows the medical tool may comprise a steerable tip 3600 disposed at the distal end of a sheath. The steerable tip 3600 may include a transducer 3602 (e.g., ultrasound transducer, folded transducer). When compared to tip 3500, the tip 3600 may include a shortened folded end 3601. Inactive piezo material may be minimized adjacent the transducer 3602. As an example, an ASIC 3604 may be disposed adjacent the transducer 3602. The tip 3600 may include various sensors, control boards, thermistors, and the like. As shown, the tip 3600 includes thermistors 3606 disposed proximal to the transducer 3602. Circuits such as flex circuits may be in electrical communication with one or more of the components in the tip 3600. As an example, the flex circuit may be divided into layers and selectively in communication with the components. As more clearly shown in FIG. 24, at least a portion of the tip 3600 may be at least partially enclosed by a first material 3800 (e.g., polymer) and at least a portion of the tip 3600 may be at least partially enclosed by a second material 3804. The first and second materials 3800, 3804 may be configured with the same or different characteristics such as differing stiffness (e.g., durometer). As further illustrated in FIG. 24, a navigation sensor 3802 may be disposed adjacent the transducer 3602. The navigation sensor 3802 may be in communication with a navigation system to provide tracking and position of the tip 3600 and to provide feedback to a user.

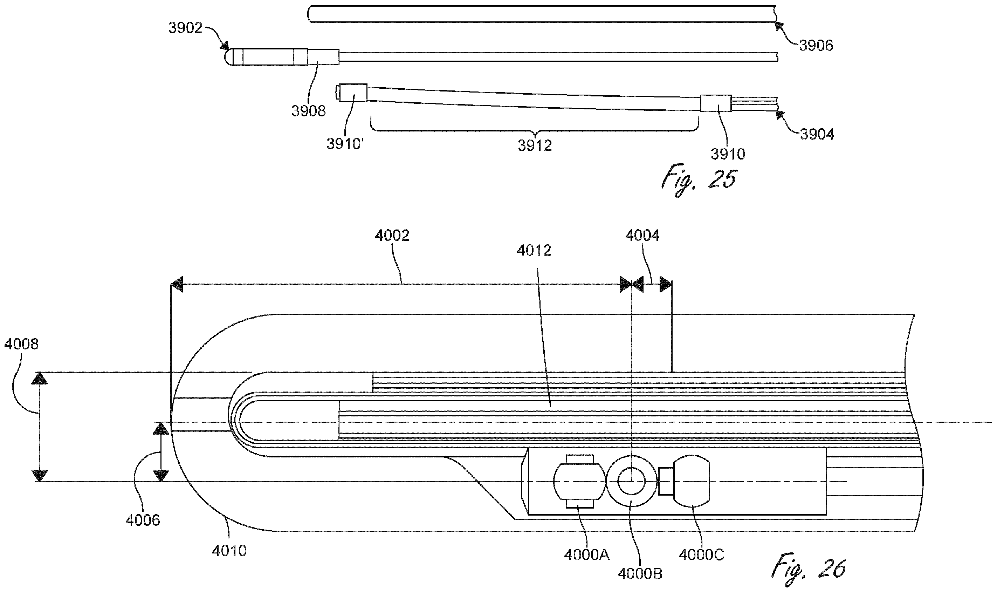

FIG. 25 illustrates a medical tool that may comprise a steerable tip 3902 disposed at the distal end of a sheath 3906. As shown, a double axis sensor (DAS) 3908 may be disposed at or adjacent a proximal end of the tip 3902. An inner sheath 3904 may comprise a section configured for deflection 3912 and may be disposed adjacent the tip 3902 and within the sheath 3906. One or more DAS sensors 3910, 3910' may be disposed along a length of the sheath 3904. One or more of the DAS sensors 3910, 3010' could alternatively be configured as SAS sensors.

FIG. 26 illustrates example configurations of a triple axis (TAS) navigation sensor 4000, comprising an x-axis coil 4000A, y-axis coil 4000B, and z-axis coil 4000C. This TAS sensor position may be facilitated by an extra cavity in the molded tip 4010 within which the TAS sensor 4000 may be bonded. The TAS location directly under the transducer 4112 provides a closer alignment to the transducer which may improve the accuracy of the ultrasound volume relative to the catheter tip when displayed in the CARTO system. For example, from a tip of the device to a center of a y-coil may be measured at a first length 4002. From the center of an active transducer 4002 to the y-coil may be a second length 4004. From a sensor axis to a tip axis may be a third length 4006. From a sensor axis to a face of an ultrasound transducer may be a fourth length 4008. The lengths may be programmed into an EEPROM in the catheter as calibration parameters that are read by the CARTO system to ensure accurate position of the Catheter in the CARTO system. The TAS sensor 4000 could alternatively be replaced with a DAS sensor with alternative calibration parameters.

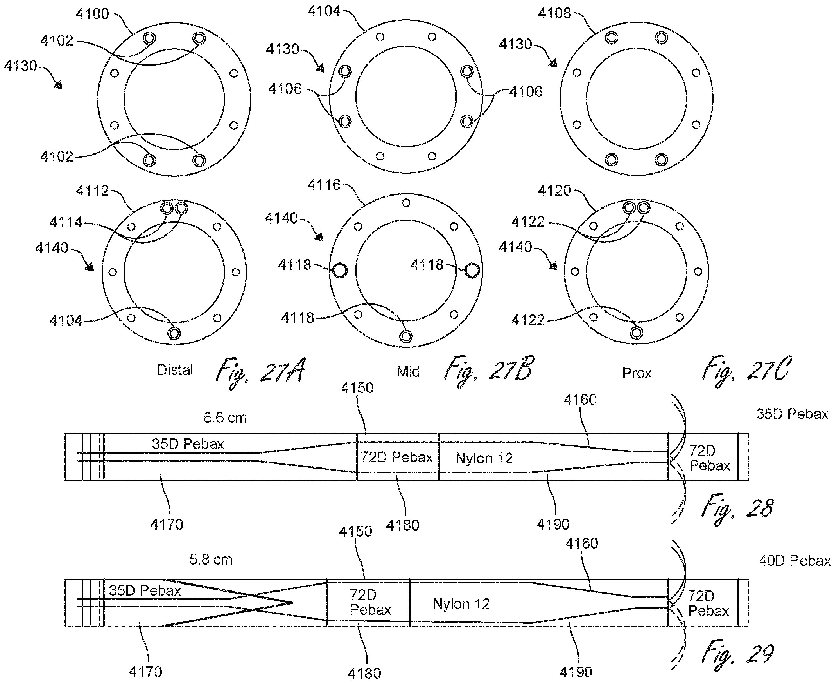

FIGS. 27A-27C illustrate various arrangements of pull wires showing a first aspect 4130 and a second aspect 4140 in vertical pairings for comparison. For example, a handle assembly may have internal components that interface with proximal ends of such pull wires such that actuation of one or more actuators tensions one or more pull wires to cause deflection of an inner shaft. As shown, for example, wires 4102 may have relative configuration within an inner shaft 4100. Wires 4106 may have relative configuration within an inner shaft 4104. Wires 4110 may have relative configuration within an inner shaft 4108. Wires 4114 may have relative configuration within an inner shaft 4112. Wires 4118 may have relative configuration within an inner shaft 4116. Wires 4122 may have relative configuration within an inner shaft 4120.

Turning to FIGS. 28-29, for example, from left to right, the configurations of an inner shaft 4150 and wires 4160 (showing the first aspect 4130 and the second aspect 4140 in vertical pairings for comparison) may represent a distal portion 4170, a mid portion 4180, and a proximal portion 4190 along a length of the inner shaft. FIGS. 28-29 illustrate pull wires 4160 may change spacing and configuration along the length on one or more shafts or sheaths. As shown, for example, the distal portion 4170, the mid portion 4180, and the proximal portion 4190 along a length of the inner shaft 4150 may have varying stiffness, as shown for illustration only. Stiffness may be achieved based on material selection, such as by using varying durometer materials (35D Pebax, 40D Pebax, 55D Pebax, 63D Pebax, 72D Pebax, Nylon 12, etc.). As a further illustration, the selection of stiffness (or other characteristics) of one or more of the portions of the shaft may be based on desired flex, straightness, and or shaping of the shaft to provide various control modes.

For a given pair of pull wires 4160, the pair may be close together in the handle assembly, diverge further apart over most of the shaft length, then come back together distally, where the shaft is intended to deflect. As an illustrative example, and as shown in FIGS. 28-29, at least a pair of the plurality of pull wires 4160 are disposed adjacent each other in parallel configuration along the longitudinal length of the deflectable shaft from the handle assembly (not shown, adjacent the proximal portion 4190) to a select bifurcation region (e.g., 4180) disposed distally from the handle assembly, wherein at least the pair of the plurality of pull wires are spaced further from each other at the bifurcation region than the handle assembly, and the at least the pair of the plurality of pull wires are spaced closer to each other in a second region (e.g., 4170) of the deflectable shaft disposed distally from the bifurcation region compared to the bifurcation region. As a further example, at least a portion of the second region exhibits a stiffness that is less than a stiffness of the bifurcation region.

Extending the non-limiting example, at least a first pair of the plurality of pull wires 4160 may be disposed adjacent each other in parallel configuration along the longitudinal length of the deflectable shaft in a first region (e.g., 4190) of the deflectable shaft to a select first bifurcation position disposed distally from the handle assembly and at least a second pair of the plurality of pull wires 4160 may be disposed adjacent each other in parallel configuration along the longitudinal length of the deflectable shaft in a second region of the deflectable shaft to a select second bifurcation position disposed distally from the handle assembly. As such, the at least the first pair of the plurality of pull wires 4160 may be spaced further from each other in a third region disposed distally from the first bifurcation point and the second pair of the plurality of pull wires 4160 may be spaced further from each other in a fourth region disposed distally from the second bifurcation point.

Additionally or alternatively, the pull wires discussed herein may be configured such that the bifurcation points of the pull wires are positioned within a stiffer region (e.g., 72D region), which may provide desired control of deflection (e.g., deflection of a portion of the shaft distal to the bifurcation point). Other configurations may be used. Additionally or alternatively, pull line convergence and bifurcation locations can be configured to be the same or different on either side of the inner shaft. As an example, where pull lines on opposite sides of the inner shaft converge or bifurcate at the same longitudinal position on each side, the deflection of the shaft may be symmetric in either direction. However, where the where pull lines on opposite sides of the inner shaft converge or bifurcate at different longitudinal positions, the deflection of the shaft in one direction may have a larger or a smaller radius of curvature and may provide asymmetrical deflection control.

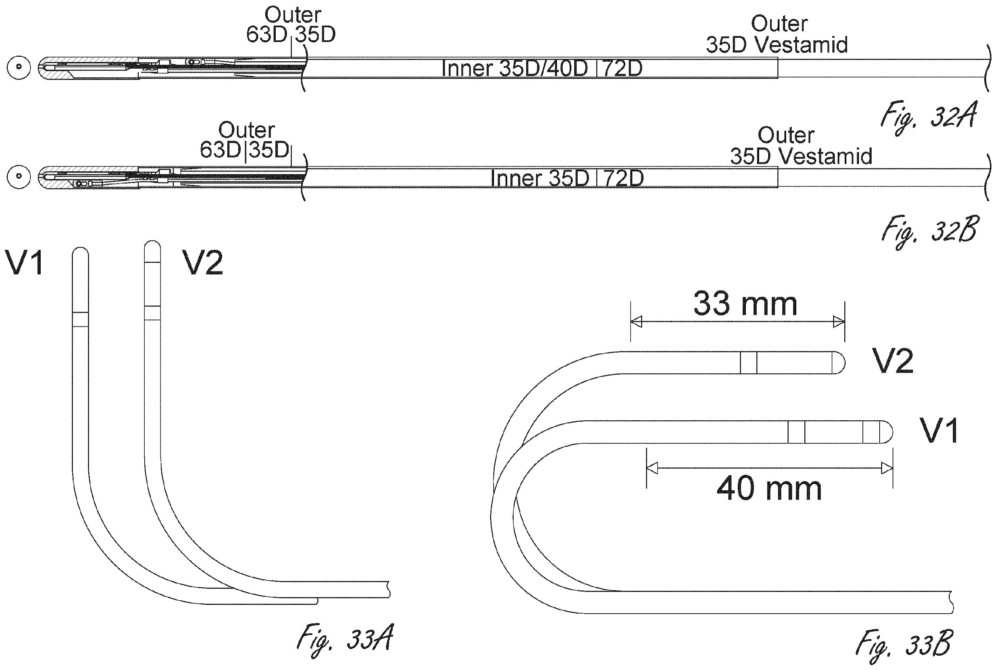

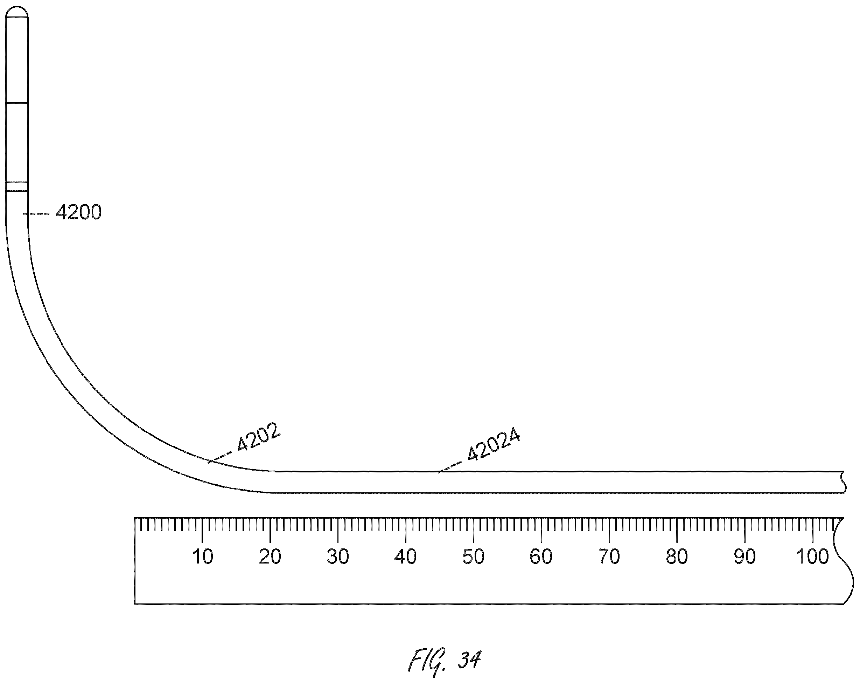

As a further example, FIGS. 32-33 illustrate how the comparative changes in stiffness and length of the first aspect and the second aspect may affect the flex and straight tip length of the shafts. As shown in FIGS. 32-33, for example, the second embodiment illustrates a straight tip length of 33mm, while the first embodiment illustrates a straight tip length of 40mm. Additionally, bifurcation of the wires (e.g., pull line) may provide additional control of the inner shaft, as shown in FIG. 34.

FIG. 34 illustrates, for example, how the configurations of the second aspect shown in FIGS. 28-29 and 32-34 allow the catheter to bend at point 4200. Additionally, the transition in the region proximal to the deflection region from a more flexible region 4202 to a stiffer region 4204 to improve maneuverability of the catheter. Such a configuration may be useful in various application, such as navigating to more distal regions in the heart. As an example, the catheter may need to cross the tricuspid valve and bend up and across to the PA valve. As the catheter traverses the heart, the catheter may need to flex through an 'S' curve to relieve excessive force transmission. The stiffness transition proximal to the deflection region may also assist the catheter as it is advanced forward in the heart. As illustrated, the configurations of the second aspect allow the catheter to bend through a turn into the right atrium from the right ventricular outflow tract. Additionally, the configurations of the second aspect may allow the catheter to maneuver in the right atrium when the tip of the catheter is in the pulmonary artery or deeper in the left atrium.

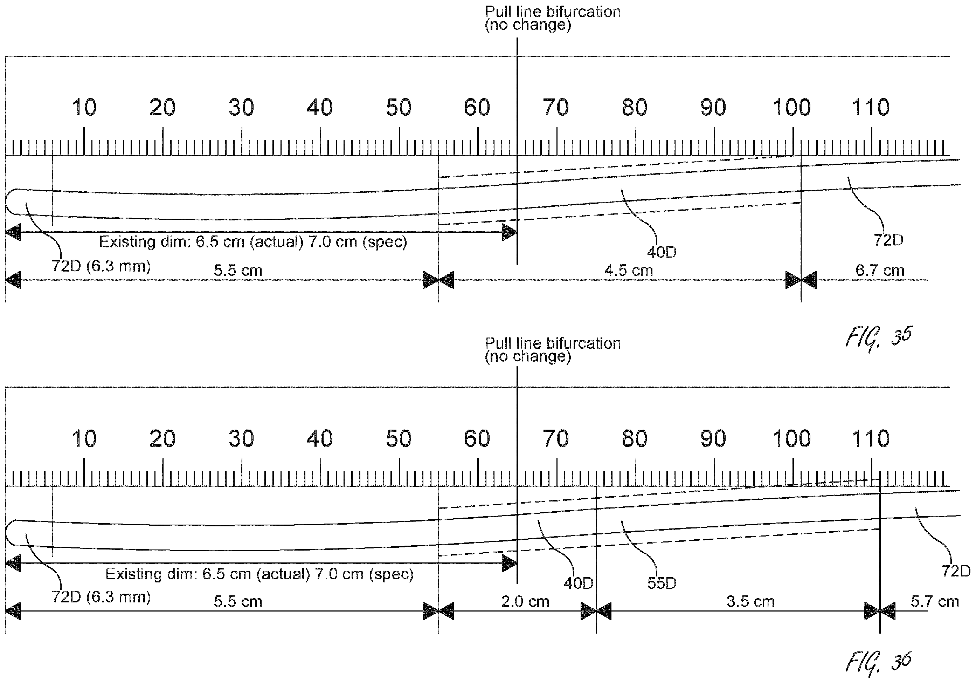

Additionally, FIGS. 35-36 illustrate comparative changes in stiffness and length of the first aspect and the second aspect of the inner shaft relative to a bifurcation point of the wires. Other variations of durometer combinations of the inner and outer shaft may be used to optimize the transition in stiffness along the approximately 15 cm distal portion of the catheter.

As an illustrative example, a symmetric design whereby pull wires are bifurcated and change relative position to each other in various sections of the shaft. By controlling the stiffness in the shaft and the positioning of the pull lines (e.g., moving a bifurcation point more distally) one may reduce off-axis curl, while providing more precise control of the deflection of the distal end of the shaft.

Disclosed herein is a medical device comprising a distal end. The distal end may comprise a single tail comprising a transducer and an application-specific integrated circuit (ASIC). The single tail may comprise multi-layer flex electrodes. The transducer and/or the ASIC may utilize methods comprising the multi-layer flex electrodes. The single tail may comprise multi-layer foil traces to facilitate high-density routing. The single tail may comprise anisotropic conductive film (ACF) bonds to accommodate electro acoustic module (EAM) (SAP) traces and ASIC lines. The multi-layer flex design may incorporate via connections to connect or isolate various layers. The connections may include blind connections, through connections, and/or buried connections. The systems and methods described herein support numerous embodiments, including single-sided multi-layer flexes or double-sided connection pads. The elimination of folding enhances robustness and reduces the overall length of the stiff section of the catheter tip, which directly improves maneuverability and access within patient anatomy.

In another embodiment, a multi-layer flex design may be used to integrate long flexes. The EAM may be manufactured with long flexes and zero insertion force (ZIF) connector at a proximal end and/or distal end for printed circuit board (PCB) connections, which may reduce touch time, reduce manufacturing complexity, and reduce overall product cost.

An advantage of the systems and methods described herein at that a multi-layer flex as described herein may allow for the capability to create individual flex assemblies for the transducer and ASIC, which may allow an acoustic stack to be diced on dedicated panels, which may mitigate the risk of damaging PCB components during a dicing process.

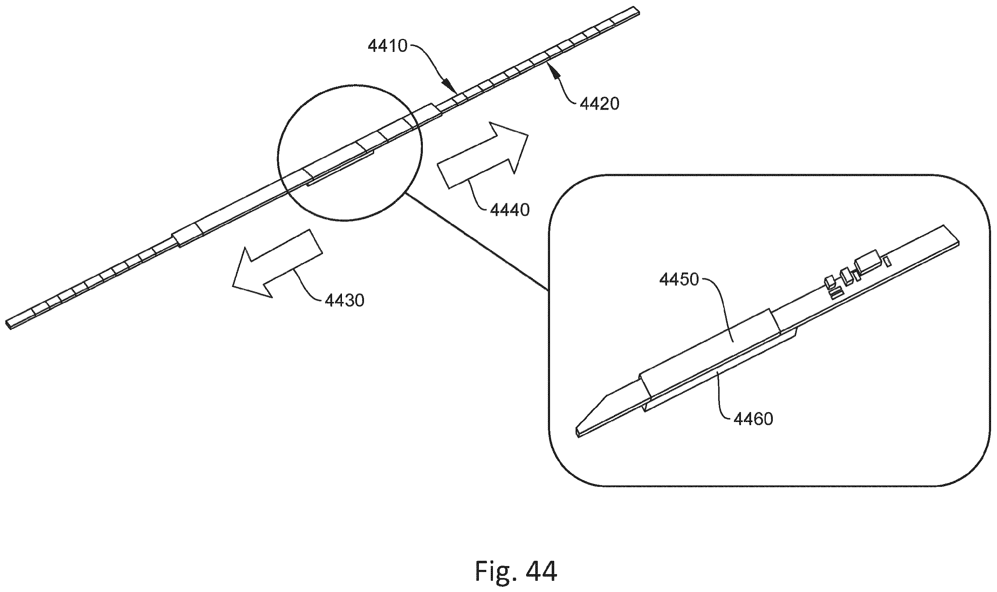

The EAM may be a primary component in a medical device, such as a catheter assembly. The EAM may reside within a medical device configuration shown in Fig. 44. The medical device configuration may comprise an ultrasound transducer attached to an integrated ASIC and flex circuits. The flex circuits may have two ends, one distal 4430 and one proximal 4440. Transducer stack layers 4450 may be mounted on top of a transducer flex 4410 and the ASIC 4460 and backing may be mounted on back of an ASIC flex 4420. An issue with this configuration is the need to fold the distal flexes 180 degrees to assemble into the EAM tip. The two-sided configuration presents multiple challenges. First, folding the distal flexes increases the risk of trace breaks due to the aggressive bend radius required for assembly. Additionally, this configuration increases the overall length of the EAM component, which extends a stiff section of a catheter. Lengthening the stiff section of the catheter limits maneuverability by shifting a deflection curve further proximal relative to the transducer.

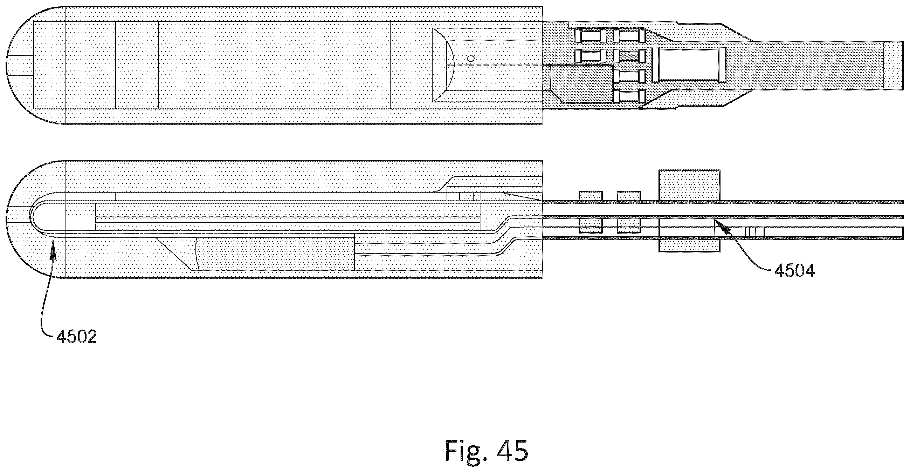

Turning to Fig. 45, a medical device configuration is shown. The medical device configuration may comprise a two-wing flex circuit design, which requires the folding of the distal tail. The medical device configuration may comprise a 180-degree fold 4502. The medical device configuration may comprise a folded distal flex tail 4504. The medical device configuration may be prone to trace breaks. The trace breaks may be due to a small bend radius. The medical device configuration may comprise a flex stack. The flex stack may be composed of two independent flex circuits, one for a transducer and one for an ASIC.

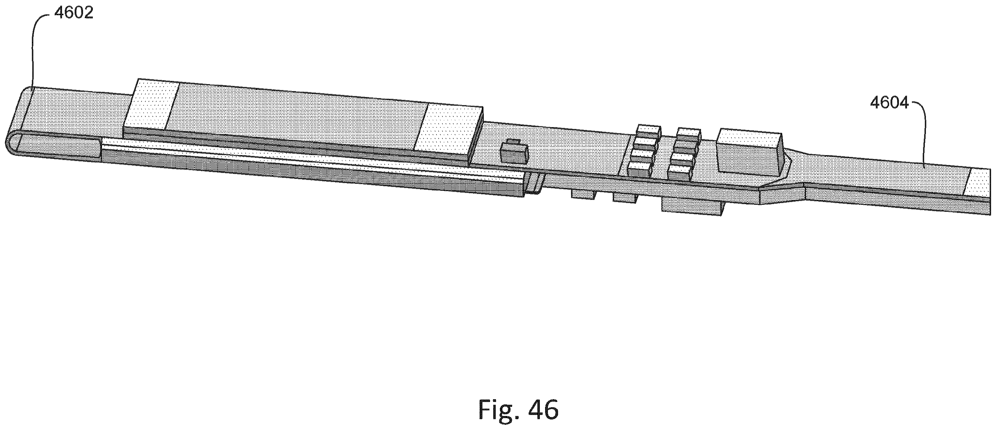

Turning to Fig. 46, a medical device configuration is shown. The medical device configuration may comprise a folded flex wing 4602. The medical device configuration may comprise a long flex 4604. The long flex 4604 may comprise an M-fold. The long flex 4604 may extend ridged length of an EAM tip.

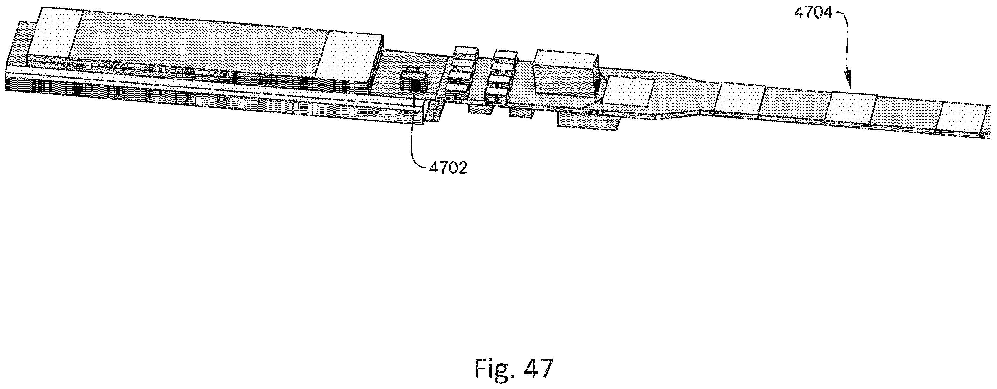

Turning to Fig. 47, a medical device configuration according to the present disclosure is shown. The medical device configuration may comprise a preloaded thermistor 4702. The medical device configuration may comprise a flex 4704. The flex 4704 may comprise a single wing design. The flex 4704 may comprise no folding. The flex 4704 may comprise an anisotropic conductive film (ACF) pad spacing and length update. The flex 4704 may comprise a double-sided short flex. The flex 4704 may according to the medical device configuration of Fig. 47 may comprise a lower manufacturing cost as compared to the long flex 4604 of the medical device configuration of Fig. 46.

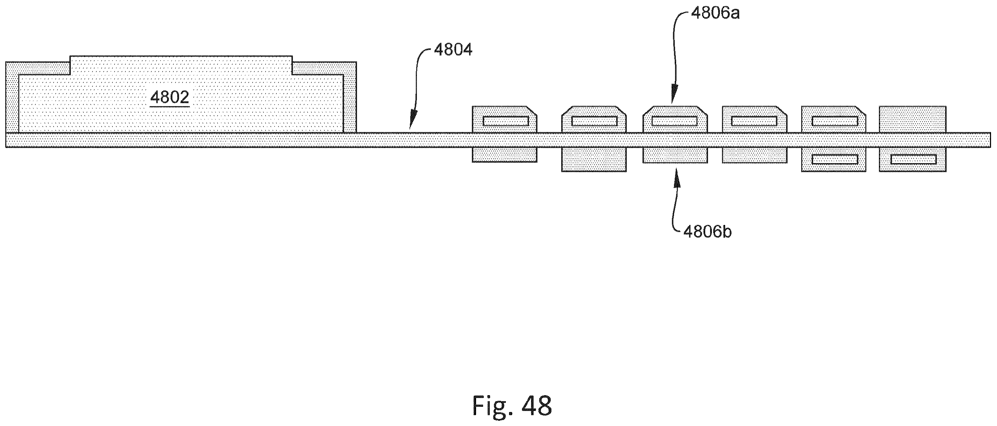

Turning to Fig. 48, a medical device configuration according to the present disclosure is shown. The medical device configuration may comprise an EAM 4802. The EAM 4802 may be mounted to a multi-layer flex 4804. The multi-layer flex 4804 may comprise double sided ACF bond pads 4806a-4806b. The medical device configuration may comprise a single tail EAM design. The medical device configuration may use multi-layer foil to route traces and ACF bond pads 4806a-4806b. Higher density multi-layer flex may allow routing of all traces distal to the EAM 4802.

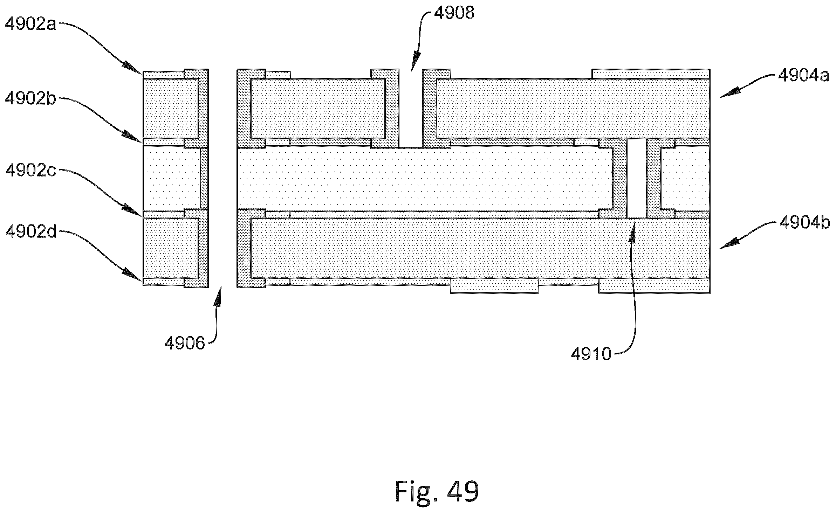

Turning to Fig. 49, a medical device configuration according to the present disclosure is shown. The medical device configuration may comprise conductive layers 4902a-d. The conductive layers 4902a-d may comprise conductive foil. The conductive foil may comprise copper. The medical device configuration may comprise insulative layers 4904a, 4904b in between the conductive layers 4902. The insulative layers 4904a, 4904b may comprise polyimide. The conductive layers 4902a-d may have a top surface 4902a and a bottom surface 4902d. The medical device configuration may comprise one or more through via (e.g., connection, etc.), such as through via 4906. A through via may extend through each of the conductive layers 4902a-d and be visible on both the top surface 4902a and the bottom surface 4902d. The medical device configuration may comprise one or more blind via (e.g., connection, etc.), such as blind via 4908. A blind via may be exposed on one of the top surface 4902a or bottom surface 4902d but ends before extending to the other surface. The medical device configuration may comprise one or more buried via (e.g., connection, etc.), such as buried via 4910. A buried via may be contained within the conductive layers such that it is not visible on either the top surface 4902a or the bottom surface 4902d. Multiple embodiments may be used to create double sided flexes using through vias, such as 4906, blind vias, such as 4908, and buried vias, such as 4910.

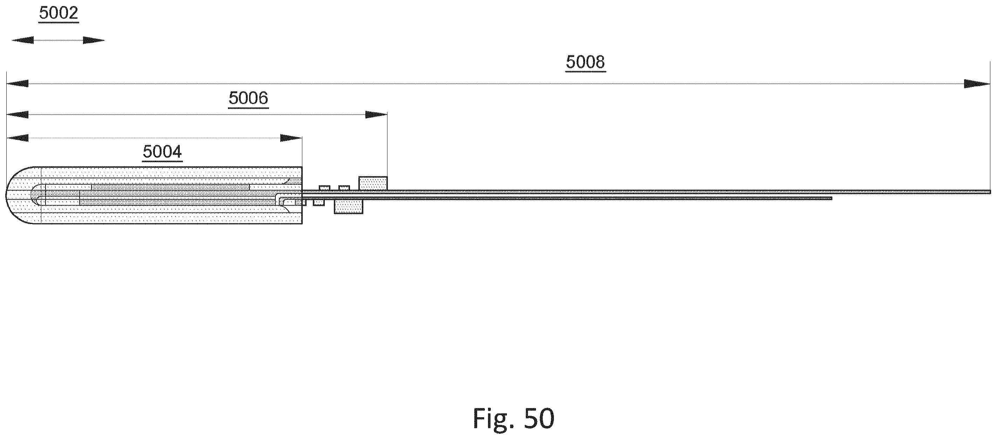

The multi-layer flex described herein prevents folding. As will be shown in reference to Figs. 50-53, eliminating the need to fold the flex allows an overall length of a stiff section of a catheter to be shorter. Having a shorter length of the stiff section of the catheter increases maneuverability of the catheter. By decreasing the stiff section, the deflection curve may be moved distal and catheter manipulation may be improved and access into a patient anatomy may be improved.

Fig. 50 shows a medical device configuration with a folded flex. The medical device configuration may have a distal fold length 5002. The medical device configuration may have a distal tip length 5004. The distal tip length 5004 may be 17 millimeters. The medical device configuration may have a stiff section length 5006. The stiff section length 5006 may be 21.9 millimeters. The medical device configuration may comprise a flex length 5008. The flex length 5008 may be 56.8 millimeters.

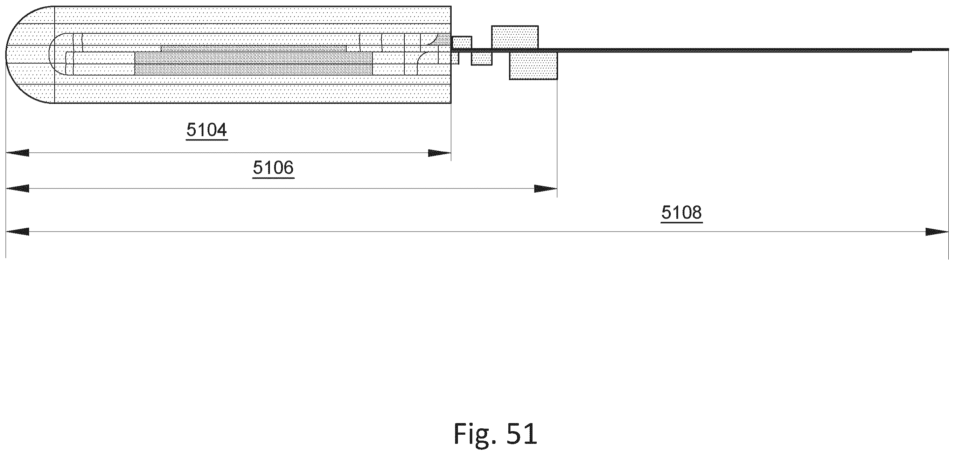

Fig. 51 shows a medical device configuration according to the present disclosure. The medical device configuration may have a distal tip length 5104. The distal tip length 5104 may be 14.7 millimeters. The medical device configuration may have a stiff section length 5106. The stiff section length 5106 may be 18.2 millimeters. The medical device configuration may comprise a flex length 5108. The flex length 5108 may be 31.2 millimeters.

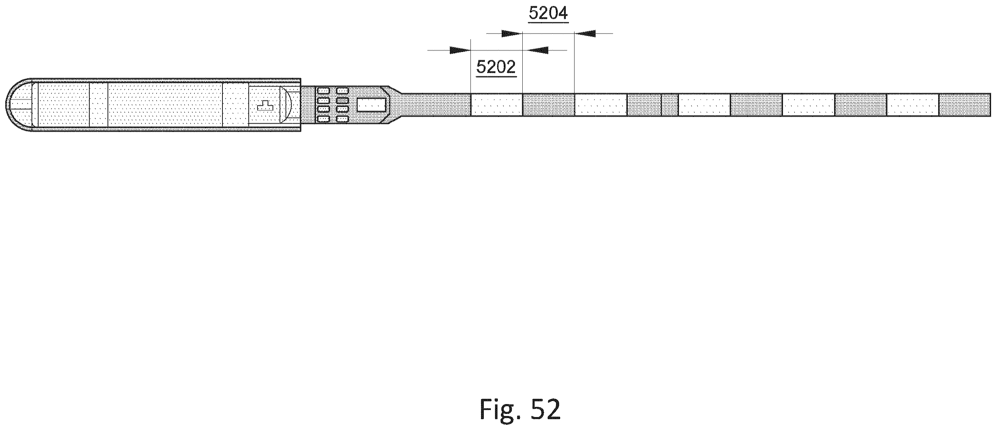

Fig. 52 shows a medical device configuration with a folded flex. The medical device configuration may comprise an ACF pad length 5202. The ACF pad length 5202 may be 3 millimeters. The medical device configuration may comprise a length of space between AFC pads 5204. The length of space between ACF pads 5204 may be 3 millimeters.

Fig. 53 shows a medical device configuration according to the present disclosure. The medical device configuration may comprise a preloaded thermistor 5302. The medical device configuration may comprise an ACF pad length 5304. The ACF pad length 5304 may be 1.2 millimeters. The medical device configuration may comprise a length of space between AFC pads 5306. The length of space between ACF pads 5306 may be 1.8 millimeters.

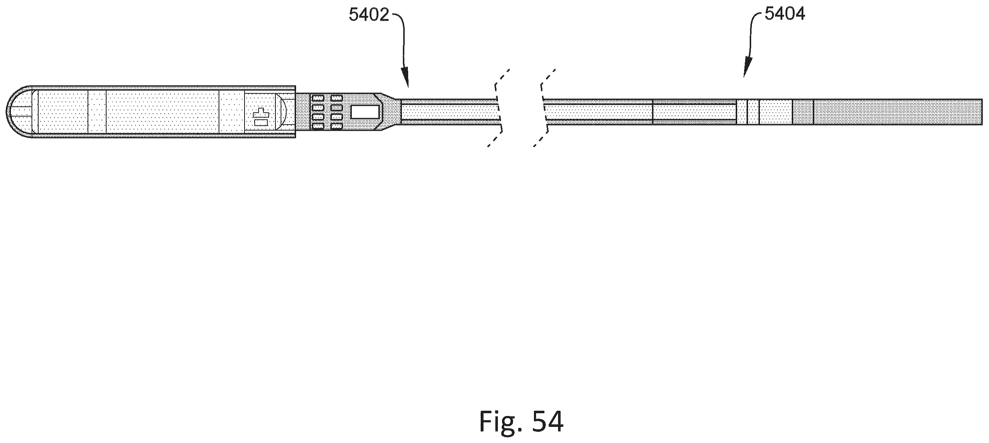

Figs. 54 shows a medical device configuration. The medical device configuration comprises an EAM connected to a long flex ACF 5402 and proximal ACF bonding 5404.

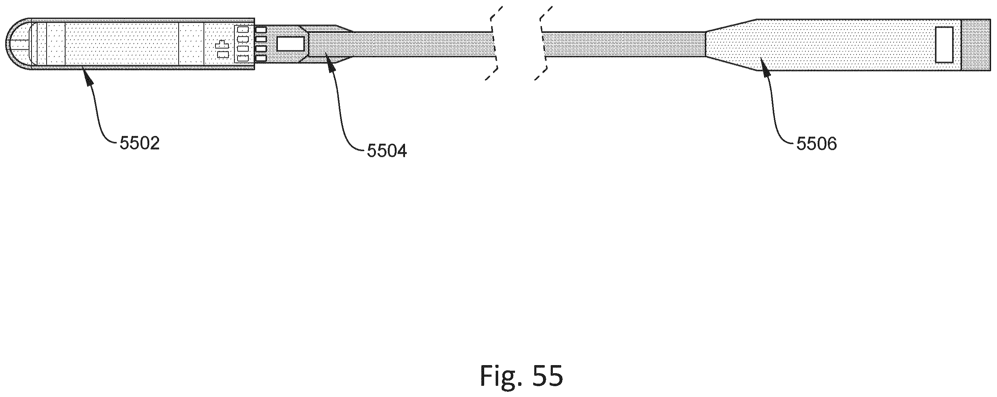

Fig. 55 shows a medical device configuration according to the present disclosure. The medical device configuration may comprise a tip 5502. As compared to the medical device configuration of Fig. 54, the tip 5502 may comprise a redesigned EAM with a shorter tip. The medical device configuration of Fig. 55 may comprise a long flex 5504. The long flex 5504 may be pre-connected into the redesigned EAM with automated sub-assembly. The medical device configuration may comprise a handle 5506. The handle 5506 may be connected to the long flex 5504 with a zero insertion force (ZIF) connector. Connection of the handle 5506 to the long flex 5504 via a proximal ZIF connection may eliminate the need to use ACF bonding to affix the handle 5506 to the long flex 5504.

The medical device configuration shown at Fig. 55 is an improvement of the medical device configuration of Fig. 54 because a long flex is integrated into an EAM in the medical device configuration of Fig. 55. The integration of the long flex into the EAM eliminates a need to ACF bond to the EAM.

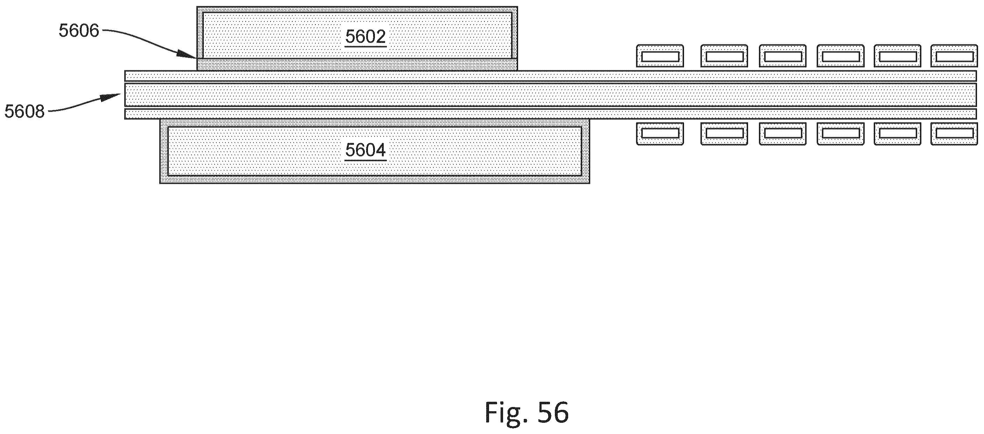

Fig. 56 shows a medical device configuration according to the present disclosure. The medical device configuration may comprise a transducer stack 5602 and an ASIC 5604. The transducer stack 5602 may be mounted (e.g., affixed, attached, etc.) to a modular transducer stack flex 5606. The modular transducer stack flex 5606 may be a separate flex assembly for the transducer stack 5602. The ASIC 5604 may be mounted to a multiple flex printed circuit board assembly (PCBA) 5608. The medical device configuration may enable a dicing process of an acoustic stack on dedicated panel. The medical device configuration may avoid concerns of dicing PCBA components and increase modularity of a manufacturing process.

The medical device configurations of Figs. 47-49, 51, 53, and 55-56 may be used in combination in various embodiments.

Regardless of the reference number with which they are labeled, any of the handle assemblies, medical tools, steerable sheaths, and electrical connections herein can be used together in a system in any combination with each other.

### EXAMPLE CLAUSES

Example Clause 1: A medical device configured and sized to be positioned within a subject, the medical device comprising: a flexible circuit comprising at least two conductive layers, wherein a first conductive layer is disposed on a first surface of the flexible circuit and a second conductive layer is disposed on a second surface opposite the first surface such that electrical energy is capable of being conducted through the flexible circuit to the first conductive layer and the second conductive layer; and an electro acoustic module (EAM) disposed at or adjacent a distal region of the flexible circuit, the EAM coupled to the flexible circuit to define a distal tip of the medical device, wherein the at least two conductive layers create trace routes via the flexible circuit to the EAM.

Example Clause 2: The medical device of Example Clause 1, wherein one or more of the at least two conductive layers comprises a conductive foil.

Example Clause 3: The medical device of Example Clause 1 or Example Clause 2, wherein the conductive foil comprises copper.

Example Clause 4: The medical device of any one of Example Clauses 1-3, wherein at least the first conductive layer and the second conductive layer are in electrical communication via at least one of a through connection, a blind connection, and a buried connection.

Example Clause 5: The medical device of any one of Example Clauses 1-4, wherein the distal tip of the medical device comprises a single tail of the flexible circuit.

Example Clause 6: The medical device of any one of Example Clauses 1-5, wherein the flexible circuit further comprises double-sided anisotropic conductive film (ACF) bond pads disposed on one or more of the first conductive layer and the second conductive layer.

Example Clause 7: The medical device of any one of Example Clauses 1-6, wherein a polyimide is disposed between at least two of the at least two conductive layers.

Example Clause 8: A medical device configured and sized to be positioned within a subject, the medical device comprising: an electro acoustic module (EAM) disposed at or adjacent a distal region of the medical device, the EAM at least partially enclosed in a material to define a distal tip of the medical device; a handle disposed at or adjacent a proximal region of the medical device and spaced from the EAM; and a flexible circuit coupling the EAM and the handle, the flexible circuit comprising at least two conductive layers, wherein a first conductive layer is disposed on a first surface of the flexible circuit and a second conductive layer is disposed on a second surface opposite the first surface such that electrical energy is capable of being conducted through the flexible circuit to the first conductive layer and the second conductive layer, and wherein the at least two conductive layers create trace routes via the flexible circuit to the EAM.

Example Clause 9: The medical device of Example Clause 8, wherein the at least two conductive layers comprises at least two layers of a conductive foil.

Example Clause 10: The medical device of Example Clause 8 or Example Clause 9, wherein the conductive foil comprises copper.

Example Clause 11: The medical device of any one of Example Clauses 8-10, wherein at least the first conductive layer and the second conductive layer are in electrical communication via at least one of a through connection, a blind connection, and a buried connection.

Example Clause 12: The medical device of any one of Example Clauses 8-11, wherein the distal tip of the medical device comprises a single tail of the flexible circuit.

Example Clause 13: The medical device of any one of Example Clauses 8-12, wherein the flexible circuit further comprises double-sided anisotropic conductive film (ACF) bond pads disposed on one or more of the first conductive layer and the second conductive layer.

Example Clause 14: The medical device of any one of Example Clauses 8-13, wherein a polyimide is disposed between at least two of the at least two conductive layers.

Example Clause 15: A medical device configured and sized to be positioned within a subject, comprising: a flexible circuit comprising at least two conductive layers, wherein the at least two conductive layers comprise a printed circuit board assembly (PCBA); an electro acoustic module (EAM) disposed at or adjacent a distal region of the flexible circuit, the EAM coupled to a first side of the flexible circuit by a material to define a distal tip of the medical device; and an application-specific integrated circuit (ASIC) disposed at or adjacent the distal region of the flexible circuit, the ASIC coupled to a second side of the flexible circuit opposite the first side.

Example Clause 16: The medical device of Example Clause 15, wherein one or more of the at least two conductive layers comprises a conductive foil.

Example Clause 17: The medical device of Example Clause 15 or Example Clause 16, wherein the conductive foil comprises copper.

Example Clause 18: The medical device of any one of Example Clauses 15-17, wherein at least a first conductive layer and a second conductive layer of the at least two conductive layers are in electrical communication via at least one of a through connection, a blind connection, and a buried connection.

Example Clause 19: The medical device of any one of Example Clauses 15-18, wherein the distal tip of the medical device comprises a single tail of the flexible circuit.

Example Clause 20: The medical device of any one of Example Clauses 15-19, wherein the flexible circuit further comprises double-sided anisotropic conductive film (ACF) bond pads disposed on one or more of a first conductive layer and a second conductive layer of the at least two conductive layers.

Example Clause 21: The medical device of any one of Example Clauses 15-20, wherein a polyimide is disposed between at least two of the at least two conductive layers.

Any of the technology, including ultrasound and steering technology, in any of the following U.S. patent references may be incorporated into any of the medical tools, devices, systems, or methods of use thereof herein, the disclosures of which are incorporated by reference herein: 6100626, 6537217, 6559389, 7257051, 7297118, 7331927, 7338450, 7451650, 7451650, 7527591, 7527592, 7569015, 7621028, 7731516, 7740584, 7766833, 7783339, 7791252, 7791252, 7819802, 7824335, 7966058, 8057397, 8096951, 8207652, 8207652, 8213693, 8364242, 8428690, 8451155, 8527032, 8659212, 8721553, 8727993, 8742646, 8742646, 8776335, 8790262, 8933613, 8978216, 8989842, 9055883, 9439625, 9575165, 9639056, and 20080287783.

The foregoing disclosure provides illustration and description but is not intended to be exhaustive or to limit the implementations to the precise form disclosed. Modifications may be made in light of the above disclosure or may be acquired from practice of the implementations. As used herein, the term "component" is intended to be broadly construed as hardware, firmware, or a combination of hardware and software. It will be apparent that systems and/or methods described herein may be implemented in different forms of hardware, firmware, and/or a combination of hardware and software. The actual specialized control hardware or software code used to implement these systems and/or methods is not limiting of the implementations. Thus, the operation and behavior of the systems and/or methods are described herein without reference to specific software code - it being understood that software and hardware can be used to implement the systems and/or methods based on the description herein. As used herein, satisfying a threshold may, depending on the context, refer to a value being greater than the threshold, greater than or equal to the threshold, less than the threshold, less than or equal to the threshold, equal to the threshold, and/or the like, depending on the context. Although particular combinations of features are recited in the claims and/or disclosed in the specification, these combinations are not intended to limit the disclosure of various implementations. In fact, many of these features may be combined in ways not specifically recited in the claims and/or disclosed in the specification.

Although each dependent claim listed below may directly depend on only one claim, the disclosure of various implementations includes each dependent claim in combination with every other claim in the claim set. No element, act, or instruction used herein should be construed as critical or essential unless explicitly described as such. Also, as used herein, the articles "a" and "an" are intended to include one or more items and may be used interchangeably with "one or more." Further, as used herein, the article "the" is intended to include one or more items referenced in connection with the article "the" and may be used interchangeably with "the one or more." Furthermore, as used herein, the term "set" is intended to include one or more items (e.g., related items, unrelated items, a combination of related and unrelated items, and/or the like), and may be used interchangeably with "one or more." Where only one item is intended, the phrase "only one" or similar language is used. Also, as used herein, the terms "has," "have," "having," or the like are intended to be open-ended terms. Further, the phrase "based on" is intended to mean "based, at least in part, on" unless explicitly stated otherwise. Also, as used herein, the term "or" is intended to be inclusive when used in a series and may be used interchangeably with "and/or," unless explicitly stated otherwise (e.g., if used in combination with "either" or "only one of").

## Claims

1. A medical device configured and sized to be positioned within a subject, the medical device comprising:
a flexible circuit comprising at least two conductive layers, wherein a first conductive layer is disposed on a first surface of the flexible circuit and a second conductive layer is disposed on a second surface opposite the first surface such that electrical energy is capable of being conducted through the flexible circuit to the first conductive layer and the second conductive layer; and
an electro acoustic module (EAM) disposed at or adjacent a distal region of the flexible circuit, the EAM coupled to the flexible circuit to define a distal tip of the medical device,
wherein the at least two conductive layers create trace routes via the flexible circuit to the EAM.

2. The medical device of claim 1, wherein one or more of the at least two conductive layers comprises a conductive foil.

3. A medical device configured and sized to be positioned within a subject, the medical device comprising:
an electro acoustic module (EAM) disposed at or adjacent a distal region of the medical device, the EAM at least partially enclosed in a material to define a distal tip of the medical device;
a handle disposed at or adjacent a proximal region of the medical device and spaced from the EAM; and
a flexible circuit coupling the EAM and the handle, the flexible circuit comprising at least two conductive layers, wherein a first conductive layer is disposed on a first surface of the flexible circuit and a second conductive layer is disposed on a second surface opposite the first surface such that electrical energy is capable of being conducted through the flexible circuit to the first conductive layer and the second conductive layer, and wherein the at least two conductive layers create trace routes via the flexible circuit to the EAM.

4. The medical device of claim 3, wherein the at least two conductive layers comprises at least two layers of a conductive foil.

5. A medical device configured and sized to be positioned within a subject, comprising:
a flexible circuit comprising at least two conductive layers, wherein the at least two conductive layers comprise a printed circuit board assembly (PCBA);
an electro acoustic module (EAM) disposed at or adjacent a distal region of the flexible circuit, the EAM coupled to a first side of the flexible circuit by a material to define a distal tip of the medical device; and
an application-specific integrated circuit (ASIC) disposed at or adjacent the distal region of the flexible circuit, the ASIC coupled to a second side of the flexible circuit opposite the first side.

6. The medical device of claim 5, wherein one or more of the at least two conductive layers comprises a conductive foil.

7. The medical device of claim 2, 4 or 6, wherein the conductive foil comprises copper.

8. The medical device of any of claims 1, 2 or 7 when dependent on claim 2, wherein at least the first conductive layer and the second conductive layer are in electrical communication via at least one of a through connection, a blind connection, and a buried connection.

9. The medical device of any of claims 3, 4 or 7 when dependent on claim 4, wherein at least the first conductive layer and the second conductive layer are in electrical communication via at least one of a through connection, a blind connection, and a buried connection.

10. The medical device of any of claims 5 to 7 when dependent on claim 6, wherein at least a first conductive layer and a second conductive layer of the at least two conductive layers are in electrical communication via at least one of a through connection, a blind connection, and a buried connection.

11. The medical device of any preceding claim, wherein the distal tip of the medical device comprises a single tail of the flexible circuit.

12. The medical device of any of claims 1, 2, 7 when dependent on claim 2, 8 or 11, wherein the flexible circuit further comprises double-sided anisotropic conductive film (ACF) bond pads disposed on one or more of the first conductive layer and the second conductive layer.

13. The medical device of any of claims 3, 4, 7 when dependent on claim 4, 10 or 11, wherein the flexible circuit further comprises double-sided anisotropic conductive film (ACF) bond pads disposed on one or more of the first conductive layer and the second conductive layer.

14. The medical device of any of claims 5, 6, 7 when dependent on claim 6, 10 or 11, wherein the flexible circuit further comprises double-sided anisotropic conductive film (ACF) bond pads disposed on one or more of a first conductive layer and a second conductive layer of the at least two conductive layers.

15. The medical device of any preceding claim, wherein a polyimide is disposed between at least two of the at least two conductive layers.
